# EUROPEAN PATENT SPECIFICATION

(11) **EP 2 692 853 B1**
(45) Date of publication and mention of the grant of the patent: **06.05.2026**
(21) Application number: 12764557.0
(22) Date of filing: 30.03.2012
(51) Int. Cl.: C12M 3/00, C12N 5/074, C12N 15/09, C12M 1/00, B01D 63/08, B01D 67/00, B01D 71/50, B05D 1/18, B05D 3/06, C12N 5/0775, C12M 1/32, C12M 1/12

(54) **MEMBRANE-SEPARATION-TYPE CULTURE DEVICE, MEMBRANE-SEPARATION-TYPE CULTURE KIT AND STEM CELL SEPARATION METHOD USING SAME**
KULTURVORRICHTUNG MIT MEMBRANTRENNUNG, KULTURKIT MIT MEMBRANTRENNUNG UND STAMMZELLENTRENNUNGSVERFAHREN DAMIT
DISPOSITIF DE CULTURE DE TYPE SÉPARATION SUR MEMBRANE, KIT DE CULTURE DE TYPE SÉPARATION SUR MEMBRANE ET PROCÉDÉ DE SÉPARATION DE CELLULES SOUCHES LES EMPLOYANT

(30) Priority: 30.03.2011 JP 2011075861
(43) Date of publication of application: 05.02.2014
(73) Proprietor: National Center for Geriatrics and Gerontology, Obu-shi, Aichi 4748511 (JP); Nepa Gene Co., Ltd., Ichikawa-City, Chiba 272-0114 (JP)
(72) Inventor: NAKASHIMA, Misako, Obu-shi, Aichi 4748511 (JP); IOHARA, Koichiro, Obu-shi, Aichi 4748511 (JP); YAMADA, Kazumasa, Obu-shi, Aichi 4748511 (JP); SHIMAGAKI, Masaaki, Urayasu-shi, Chiba 2798555 (JP); OSABE, Masahiro, Otsu-shi Shiga 520-8558 (JP)
(74) Representative: Kador & Partner Part mbB
(86) International application number: PCT/JP2012/058637
(87) International publication number: WO 2012/133803

(56) References cited:
- WO-A1-2009/113733
- WO-A1-2009/123088
- US-A- 4 720 343
- US-A1- 2004 091 397
- US-A1- 2009 142 835
- KUCIA M. ET AL., ARCH. IMMUNOL. THER. EXP., vol. 54, 2006, pages 121 - 135, XP019200151
- WANG H. ET AL., EUR. J. PHARMACOL., vol. 599, 2008, pages 24 - 31, XP025612096
- SHIN'ICHI OSHIMA ET AL.: "Ha Enmei-ka o Mezasu Shizui Saisei Jitsuyoka no Airo Kaisho", KAGAKU GIJUTSU SYSTEM KAIKAKU JIGYO HEISEI 22 NENDO SHINKI SAITAKU KADAI PRESS KOHYO SHIRYO, 1 February 2011 (2011-02-01), XP055142369, Retrieved from the Internet <URL:http://www.jst.go.jp/shincho/pdf/h22press/201015702010pa.pdf> [retrieved on 20120627]

## Description

### Technical Field

The present invention relates to a membrane separation culture device and to a membrane separation culture kit, which may be used to separate the stem cells and dental pulp stem cells of an organism of any species, and a method for separating stem cells using the same. In particular, the present invention relates to a membrane separation culture device and a membrane separation culture kit, which are used to separate dental pulp stem cells or mesenchymal stem cells with which the root canal is to be filled for regeneration of the dental pulp, and a method for separating stem cells using the same. A separation membrane useful for the membrane separation culture device of the invention, and production of the separation membrane using a surface modification method are also disclosed. The present invention uses a separation membrane, in which adhesion of cells to the membrane upon separation of the cells by permeation is suppressed by hydrophilizing a membrane consisting of a hydrophobic polymer without impairing separation performance. Accordingly, the present invention is preferably used in the fields of separation and purification of cells including blood purification field or regenerative medicine as typical examples. Moreover, by such a polymer surface modification method, only the surface of a polymer can be simply modified, and sterilization can be simultaneously carried out. Hence, when compared with conventional methods, the polymer surface modification method can contribute to production efficiency.

### Background Art

At present, dental pulp stem cells used in biological root canal fillers for treatments such as treatment by extirpation of the pulp or the treatment of the infected root canal are fractions that are excellent in terms of angiogenic ability, nerve regeneration ability and dental pulp regeneration ability. Dental pulp-derived CD31⁻SP (side population) cells, CD105⁺ cells, or CXCR4⁺ cells, have been mainly used. SP cells are labeled with Hoechst33342, and a fraction that highly emits this pigment is then separated by flow cytometer using Hoechst Blue and Hoechst Red. Large quantities of stem cells are contained in this fraction. However, since Hoechst33342 is a DNA-binding pigment and it essentially requires the use of flow cytometer, it is said that Hoechst33342 is problematic in terms of the safety of cells.

On the other hand, in the case of using an antibody against a stem cell-specific membrane surface antigen, a method of using magnetic beads without using a flow cytometer has been developed. For example, as bone marrow stem cells, CD34 or CD133 antibody beads have been known. This method requires the use of considerable quantities of tissues or cells. Thus, if such tissues or cells are separated from dental pulp tissues, this method is inappropriate. In addition, in the case of human dental pulp, CD34-positive cells, and CD133-positive cells are hardly present in dental pulp test cells (0.01% and 0.5%, respectively), and thus, the existing (commercially available) magnetic beads methods are not appropriate. Since CD105 or CXCR4 antibody beads must be prepared to order, they may be extremely expensive. Moreover, as a device for separating stem cells from adipose tissues, Celution 800/CRS system that is based on cell separation according to enzymatic digestion or centrifugation has already been used in clinical sites. However, this device requires large quantities of tissues, cells are obtained as a heterogeneous cell group containing large quantities of precursor cells, and it is also expensive. Furthermore, as a device for separating stem cells from bone marrow tissues, Bone Marrow MSC Separation Device has been commercialized. It is considered that this device is able to collect stem cells in a short time (20 minutes) by trapping bone marrow mesenchymal cells with fibers consisting of rayon and polyethylene. This device is relatively inexpensive, but it requires relatively large quantities of bone marrow tissues (spinal fluid) and the obtained cells are of a heterogeneous cell group containing large quantities of precursor cells. Hence, a device for separating stem cells from solid tissues without using enzymatic digestion and amplifying them has not yet been developed.

Cellculture Insert (Polycarbonate Membrane Transwell (registered trademark) Inserts; 2 x 10⁵ pores/cm², pore size: 8 µm, diameter of bottom surface: 6.4 mm, diameter of opening portion: 11.0 mm, height: 17.5 mm) (Corning) used as an upper structure, can be inserted into a 24-well plate (diameter: 15.0 mm, diameter of opening portion: 15.0 mm, height: 22.0 mm) (Falcon) used as a lower structure, and the thus prepared device can be used as a membrane separation device. However, since large quantities of cells adhere to a PET membrane or a polycarbonate membrane, migration of the cells to a lower layer cannot be carried out efficiently. Further, since this device has an open shape, it has a high risk of being contaminated by microorganisms, and thus the safety of cells cannot be guaranteed.

Accordingly, it is necessary to develop a method for inexpensively, safely and efficiently separating stem cells from human dental pulp tissues or human dental pulp cells.

To date, it has been known that CXCR4⁺ cells, C-MET⁺ cells, or LIF-R⁺ cells, which are present in the bone marrow, can be each concentrated to the stem cells of the bone marrow as a result of migration chemotaxis effect by utilizing the concentration gradient of their ligand SDF1, HGF, or LIF (NPL 1).

It has also been known that the stem cells of the bone marrow, peripheral blood and cord blood (hematopoietic stem cells of CXCR4⁺/ lin⁻/CD133⁺/CD45⁺ cells, and mesenchymal stem cells of CXCR4⁺/lin⁻/CD133⁺/CD45⁻ cells) can also be concentrated by the concentration gradient of SDF1 in the same manner as described above (NPL 2). In this case, SDF-1 is placed in a lower chamber of a filter with a pore diameter (pore size) of 5 µm in a commercially available Costar Transwell 24-well, and cells to be concentrated are placed in an upper portion thereof. However, in view of safety, SDF-1 has not yet received pharmaceutical approval, and thus, it has been desired to develop a safe and effective migration factor that can be substituted for SDF-1.

Platelet-derived sphingosine-1-phosphate (S1P) has been known as a factor effective for migration of bone marrow stem cells (NPL 3), and protocatechuic acid has been known as a factor effective for migration of adipose stem cells (NPL 4). However, problems regarding safety have not yet been solved. Moreover, it has been known that dental pulp CD31⁻SP cells excellent in terms of angiogenic ability, nerve regeneration ability and dental pulp regeneration ability migrate towards SDF-1 or VEGF (NPL 5). However, at present, migration factors effective for separation of dental pulp stem cells excellent in terms of angiogenic ability, nerve regeneration ability and dental pulp regeneration ability have not been discovered, other than SDF-1.

A membrane separation culture device and a migration factor, which are capable of separating dental pulp stem cells safely and practically, are required. In addition, in biological studies, it has been desired to clarify a mechanism of inducing differentiation of stem cells into various types of tissues, not only in human beings but also in various organism species. With progression of regenerative medicine, it has been strongly desired to develop a membrane separation culture device and a migration factor, which are capable of separating stem cells simply and stably.

A medical separation membrane that is brought into contact with body fluid, blood or cells has many problems to be solved. For example, when proteins, platelets, or cells adhere to such a medical separation membrane, they cause a reduction in the performance of the separation membrane or vital reactions, and also promote adsorption of the cells. In addition, in the case of water-treating membranes used in water purifiers, the adhesion of proteins or organic substances causes a reduction in the performance of such a separation membrane. In order to solve such a problem, an attempt has been made to hydrophilize the separation membrane, and various studies have been conducted. For instance, a method which comprises mixing polyvinyl pyrrolidone as a hydrophilic polymer into a membrane consisting of polysulfone at the stage of the formation of the membrane and then molding the mixture, so as to impart hydrophilicity to the membrane and to suppress contamination (PTL 1). However, in order to impart hydrophilicity to the surface of the membrane, this method is subjected to various restraints; for example, it is necessary to increase the amount of a hydrophilic polymer used in a stock solution for membrane formation, the hydrophilic polymer is limited to that compatible with a polymer used as a base material, and the optimal composition of a stock solution should be determined depending on the intended use of the material.

In addition, PTL 2 discloses a method of coating a membrane with polyvinyl acetal diethylamino acetate and a hydrophilizing agent to hydrophilize the membrane. In this method, there is a concern that polyvinyl acetal diethylamino acetate may cover the hydrophilizing agent and effects regarding non-adhesion may be significantly reduced. Moreover, there is another concern that the separation performance of a membrane may be reduced because the membrane is immersed both in a solution of the polyvinyl acetal diethylamino acetate and in a solution of the hydrophilizing agent.

There are disclosed a method which comprises water-insolubilizing a hydrophilic component, such as polyvinyl pyrrolidone, with a high-energy beam, and then introducing the resulting hydrophilic component into the formed membrane (PTL 3), and a method which comprises allowing a polysulfone separation membrane to come into contact with a solution of a hydrophilic polymer such as polyvinyl pyrrolidone, and then forming an insolubilized coating layer by radiation crosslinking (PTL 4). However, these methods have been problematic in that, since such an aqueous polymer such as polyvinyl pyrrolidone and a polysulfone polymer have a low intermolecular interaction, it is difficult to form a coating layer.

In order to solve the aforementioned problem, there has been disclosed a method which comprises allowing a polyvinyl alcohol aqueous solution having a certain range of saponification degree to come into contact with a polysulfone separation membrane, so as to efficiently form a coating layer on the surface of the membrane as a result of a hydrophobic interaction between polysulfone and vinyl acetate (PTL 5). However, this publication does not describe a method of suppressing adhesion. Thus, as a result of studies conducted by the present inventors, it was found that, if a separation membrane is simply coated with polyvinyl alcohol, the performance of the separation membrane is significantly reduced. Also, it has been known that a hydroxyl group of polyvinyl alcohol tends to activate a complement when it comes into contact with blood.

Furthermore, it is said that even if the surface of a material is coated with a hydrophilic polymer such as polyvinyl pyrrolidone or polyethylene glycol, the effect of suppressing adhesion can be obtained only temporarily (NPL 6). That is to say, a separation membrane module, in which blood compatibility is satisfied with a high-performance separation membrane, has not yet been established.
PTL 6 refers to a material (filter) for separating stem cells. The focus of the document is to provide a material having specific density K, mesh opening and fiber diameters and in particular the used material (filter) should be able to capture the stem cells.
PTL 7 relates to hydrophobic membrane made of synthetic polymer. It is an object of the disclosure to provide membranes with pronounced hydrophilic properties, i.e. which are capable of absorbing considerable amounts of water, relative to their total weight. The polymer comprises a polymer mixture of polyvinylpyrrolidone and polysufone.

### Citation List

### Patent Literature

PTL 1: Japanese Patent Publication No. 2-18695
PTL 2: Japanese Patent Laid-Open No. 8-131791
PTL 3: Japanese Patent Publication No. 8-9668
PTL 4: Japanese Patent Laid-Open No. 6-238139
PTL 5: Japanese Patent Laid-Open No. 2006-198611
PTL 6: US 2009/142835 A1
PTL 7: US 4,720,343 A

### Non Patent Literature

NPL 1: Kucia M., et al., Arch. Immunol. Ther. Exp. 2006
NPL 2: Baumert B. et al., Folia Histochemica Et Cytobiologica 2008
NPL 3: Meriane M., et al., Stem Cells 2006
NPL 4: Wang H., et al., Eur. J. Pharmacol. 2008
NPL 5: Iohara K. et al., STEM CELLS Volume 26, Issue 9, pages 2408-2418, September 2008
NPL 6: Medicinal Nanotechnology, Kyorin Tosho, pp. 115-116

### Summary of Invention

### Technical Problem

Conventional flow cytometry and antibody-coated magnetic beads method cannot be safe, highly-efficient and inexpensive separation methods, which satisfy standards used in clinical sites (Good Manufacturing Practice (GMP)). The present invention has been completed to solve the aforementioned problems, and it is an object of the present invention to provide: a membrane separation culture device capable of obtaining desired stem cells even from small quantities of tissues safely, highly efficiently and inexpensively, without using the conventional flow cytometry or antibody-coated magnetic beads in dental pulp regenerative medicine and other regenerative medicines; and a method for separating stem cells. Further, it is another object of the present invention to provide a membrane separation culture device, a membrane separation culture kit, and a method for separating stem cells, which can be applied to separation of stem cells of all organism species (non-human embryonic stem cells, iPS cells, and tissue stem cells).

A further object of the present invention is to provide a membrane separation culture device using a high-performance separation membrane, in which the disadvantages of prior art regarding such a separation membrane have been improved, which has compactness sufficient for being used in ordinary cell culture incubators or clean benches, and in which not only filtration performance caused by pore diameter but also surface affinity has been improved.

### Solution to Problem

The present inventors have conducted intensive studies directed towards achieving the aforementioned objects. As a result, the inventors have found that stem cells are allowed to selectively pass from the upper portion of a membrane to a lower portion thereof using the concentration gradient of a cell migration factor, so that the stem cells can be separated, thereby completing the present invention. Thus, according to one aspect, the present invention relates to a membrane separation culture device comprising: an upper structure constituted with a vessel in which at least a portion of the bottom thereof is formed with a separation membrane having pores that allow stem cells to permeate therethrough; and a lower structure constituted with a vessel that retains a fluid in which the separation membrane of the upper structure is immersed, wherein the separation membrane comprises: a base material membrane consisting of a hydrophobic polymer; and a functional layer formed by allowing one or more hydrophilic polymers selected from a vinyl pyrrolidone polymer, a polyethylene glycol polymer and a vinyl alcohol polymer to bind to the surface of the base material membrane via a covalent bond; wherein the weight percentage of the hydrophilic polymer(s) constituting the functional layer is 1.5% to 35% based on the total weight of the separation membrane, and the size of the pore is 3 µm to 10 µm and the pore density is 1 × 10⁵ to 4 × 10⁶ pores/cm².

It is preferable that the membrane separation culture device comprise a plurality of the upper structures, and further comprise a frame body that is accommodated in the lower structure and comprises a plate-like member in which a plurality of holes are each established to lock the plurality of the upper structures.

Alternatively, it is preferable that the membrane separation culture device comprise a plurality of the upper structures, and further comprise a frame body that is accommodated in the lower structure and comprises a plate-like member in which a plurality of holes are each established to lock the plurality of the upper structures, and that the lower structure be constituted with a plurality of vessels each corresponding to the plurality of the upper structures.

It is a preferred embodiment according to claim 2 that the plurality of the upper structures have separation membranes each having a different pore size and/or a different pore density.

It is preferable that the membrane separation culture device further comprise a lid structure for covering or hermetically sealing the upper structure and the lower structure.

It is preferable that the lid structure further comprises a gas exchanger comprising a gas inlet port and a gas discharge port.

It is preferable that at least a portion of the lid structure be formed with a membrane having pores whose pore size is 1 to 100 nm.

It is preferable that a hermetic sealing elastic body be established between the lid structure and the lower structure.

It is preferable that the membrane separation culture device further comprise a temperature control system containing a temperature-measuring device and a temperature-controlling device.

It is preferable that the lower structure further comprise a medium replacement system comprising a medium inlet port and a medium outlet port.

The present invention relates to a membrane separation culture kit as defined in claim 3 comprising the membrane separation culture device as defined in claim 1 and cell migration factor(s) to be poured into the lower structure, wherein the cell migration factor(s) are one or more selected from SDF-1, G-CSF, bFGF, TGF-β, NGF, PDGF, BDNF, GDNF, EGF, VEGF, MMP3, Slit, GM-CSF, LIF, HGF, and serum.

It is preferable according to claim 4 that the concentration of the cell migration factor(s) be 1 ng/ml to 500 ng/ml.

It is preferable according to claim 5 that the kit further comprises serum to be poured into the lower structure, and that the cell migration factor is G-CSF or bFGF.

The present invention also relates to a method for separating stem cells as defined in claim 6 using the membrane separation culture device according to the invention, wherein the method comprises: a step of dispersing test cells or test tissues on the separation membrane of the upper structure; a step of filling the vessel as a lower structure with a medium containing cell migration factor(s); and a step of allowing the separation membrane of the upper structure to come into contact with the medium in the lower structure, wherein the cell migration factor(s) is one or more selected from SDF-1, G-CSF, bFGF, TGF-β, NGF, PDGF, BDNF, GDNF, EGF, VEGF, MMP3, Slit, GM-CSF, LIF, HGF, and serum.

It is preferable according to claim 7 that the concentration of the cell migration factor(s) is 1 ng/ml to 500 ng/ml.

It is preferable that the test cells are dispersed at a density of 3 × 10² cells to 3 × 10⁴ cells per mm² of the separation membrane.

In the above described method for separating stem cells, the stem cells preferably are dental pulp stem cells, the cell migration factor preferably is G-CSF or bFGF, the concentration of the G-CSF or bFGF preferably is 50 to 150 ng/ml, the test cells are dispersed preferably at a density of 3 × 10² to 1.5 × 10³ cells per mm² of the separation membrane, or the test tissues are left at rest at a density of 0.1 mg to 1 mg per mm² of the separation membrane, and serum is added to a medium containing the cell migration factor at a volume percentage preferably of 5% to 20% based on the volume of the medium.

In the above described method for separating stem cells, the stem cells preferably are bone marrow stem cells or adipose stem cells, the cell migration factor preferably is G-CSF or bFGF, the concentration of the G-CSF or bFGF preferably is 50 to 150 ng/ml, the test cells are dispersed at a density of preferably 3 × 10² to 1.5 × 10³ cells per mm² of the separation membrane, or the test tissues are left at rest at a density of 0.1 mg to 1 mg per mm² of the separation membrane, and serum is added to a medium containing the cell migration factor at a volume percentage of preferably 5% to 20% based on the volume of the medium.

The present inventors have further conducted intensive research directed towards achieving the aforementioned object regarding a separation membrane. As a result, the inventors have found that the separation membrane and separation membrane module used in the present invention, which are excellent in terms of blood compatibility and have small amounts of proteins or organic substances adhering thereto, can be achieved with the following configurations.

Specifically, the present invention uses a separation membrane comprising: a base material membrane consisting of a hydrophobic polymer; and a functional layer formed by allowing one or more hydrophilic polymers selected from a vinyl pyrrolidone polymer, a polyethylene glycol polymer and a vinyl alcohol polymer to bind to the surface of the base material membrane via a covalent bond; wherein the weight percentage of the hydrophilic polymer(s) constituting the functional layer is 1.5% to 35% based on the total weight of the separation membrane, wherein the base material membrane has pores with a pore size of 3 to 10 µm and the density of the pore is 1 × 10⁵ to 4 × 10⁶ pores/cm² and be used for cell separation.

It is preferable that the hydrophobic polymer be selected from the group consisting of a sulfone polymer, an amide polymer, a carbonate polymer, an ester polymer a urethane polymer, an olefin polymer, and an imide polymer.

It is preferable that the separation membrane according to the present disclosure be used for separating cells by permeation.

The separation membrane used in the present invention may be produced by a method, which comprises: an immersion step of immersing a base material membrane consisting of a hydrophobic polymer having a water absorption percentage of 2% or less in a treating aqueous solution containing one or more hydrophilic polymers selected from a vinyl pyrrolidone polymer, a polyethylene glycol polymer and a vinyl alcohol polymer at a concentration of 10 to 2000 ppm, and also containing a 0.01% to 0.2% alcohol; and a modification step of irradiating the base material membrane with a high-energy beam to modify the surface of the membrane to have a protein adhesion-suppressing property and a cell adhesion-suppressing property.

Alternatively, the separation membrane used in the present invention may be produced by a method, which comprises: an immersion step of immersing a base material membrane consisting of a hydrophobic polymer having a water absorption percentage of more than 2% in a treating aqueous solution containing one or more hydrophilic polymers selected from a vinyl pyrrolidone polymer, a polyethylene glycol polymer and a vinyl alcohol polymer at a concentration of 10 to 2,000 ppm; and a modification step of irradiating the base material membrane with a high-energy beam to modify the surface of the membrane to have a protein adhesion-suppressing property and a cell adhesion-suppressing property.

It is preferable that the hydrophobic polymer be selected from the group consisting of a sulfone polymer, an amide polymer, a carbonate polymer, an ester polymer, a urethane polymer, an olefin polymer, and an imide polymer.

Herein disclosed is a method for modifying the surface of a molded body, which comprises: an immersion step of immersing a molded body having a water absorption percentage of 2% or less in a treating aqueous solution containing one or more hydrophilic polymers selected from a vinyl pyrrolidone polymer, a polyethylene glycol polymer and a vinyl alcohol polymer at a concentration of 10 to 2,000 ppm, and also containing a 0.01% to 0.2% alcohol; and a modification step of irradiating the molded body with a high-energy beam to modify the surface of the molded body to have a protein adhesion-suppressing property and a cell adhesion-suppressing property.

Alternatively disclosed herein is a method which comprises: an immersion step of immersing a molded body having a water absorption percentage of more than 2% in a treating aqueous solution containing one or more hydrophilic polymers selected from a vinyl pyrrolidone polymer, a polyethylene glycol polymer and a vinyl alcohol polymer at a concentration of 10 to 2000 ppm; and a modification step of irradiating the molded body with a high-energy beam to modify the surface of the molded body to have a protein adhesion-suppressing property and a cell adhesion-suppressing property.

The separation membrane used in the present invention may be produced using these methods for modifying a molded body.

### Advantageous Effects of Invention

According to the present invention, stem cells can be separated even from small quantities of cells safely highly efficiently and inexpensively, using a membrane separation culture device and cell migration factor(s). In addition, permeation pores with a suitable size are selected depending on the size of cells, so that the present membrane separation culture device can be used to separate the stem cells of all organism species. Otherwise, by selecting suitable cell migration factor(s), the present membrane separation culture device can be applied to separation of various types of stem cells including non-human embryonic stem cells, iPS cells, and tissue stem cells. Moreover, by changing the number of separation membranes in membrane separation culture, the present membrane separation culture device can also be applied to various amounts of tissues or cells. By adopting completely-sealed-type upper structure and lower structure, it becomes possible to separate stem cells, which comply with GMP and can be practically used in clinical sites. Furthermore, the membrane separation culture device of the present invention can be broadly used for both experimental use and clinical use, and greatly contributes to the development of regenerative medicine. Membrane-separated cells are further advantageous in that, in particular, stem cells separated by a membrane from middle-aged and elderly people have a small level of phenotypical change associated with amplification. Since such stem cells hardly become senescent and are hardly aged together with amplification, they can be effectively functional in clinical use. Moreover, a membrane separation device is further advantageous in that stem cells can be separated not only from cells but also from tissues, without previously dispersing the cells by enzymatic digestion, which leads to a reduction in the time required for such enzymatic digestion and the guarantee of safety by nonuse of enzymes.

Furthermore, the separation membrane used in the present invention intends to suppress a decrease in separation efficiency caused by adhesion of cells during cell separation, and the present separation membrane is characterized in that a polymer is localized on the surface of the functional layer of the separation membrane. The present separation membrane can be preferably used to suppress protein or cell adhesion on the surface of a separation membrane that has been molded without mixing a hydrophilic polymer into a stock solution for membrane formation, for example, on the surface of a membrane on which pores have been formed by irradiation of an electron beam.

### Brief Description of Drawings

[Fig. 1]

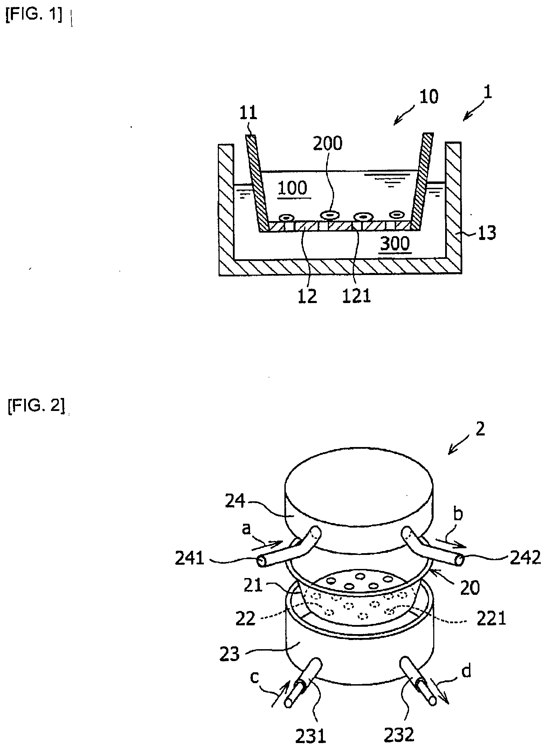

   Fig. 1 is a schematic view showing a membrane separation culture device according to a first embodiment.
[Fig. 2]
   Fig. 2 is a schematic view showing a membrane separation culture device according to a second embodiment.
[Fig. 3]

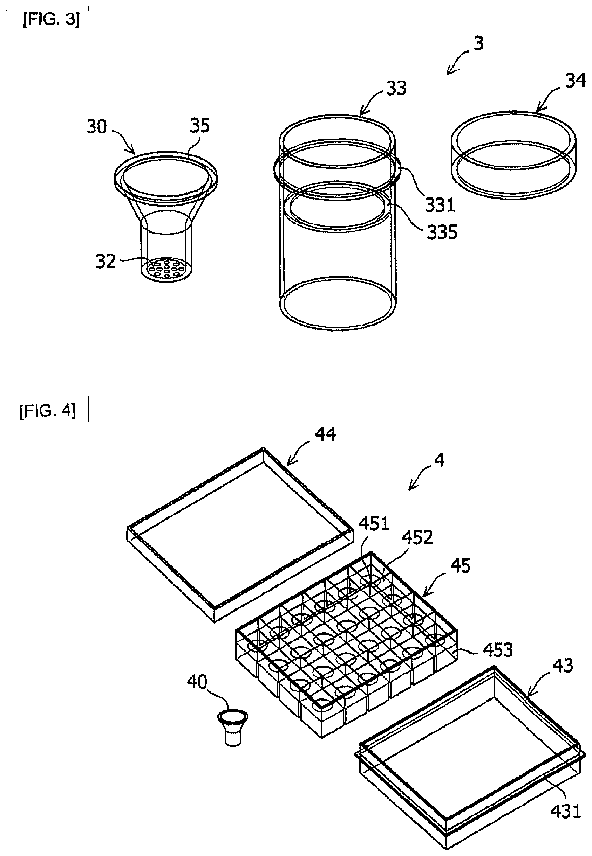

   Fig. 3 is a schematic view showing a configuration of a membrane separation culture device according to a third embodiment.
[Fig. 4]
   Fig. 4 is a schematic view showing a configuration of a membrane separation culture device according to a fourth embodiment.
[Fig. 5]

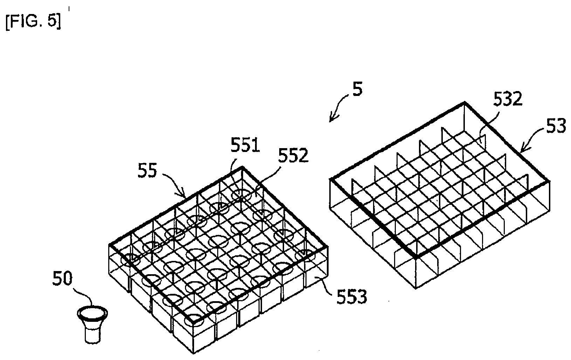

   Fig. 5 is a schematic view showing a configuration of a membrane separation culture device according to a fifth embodiment.
[Fig. 6]

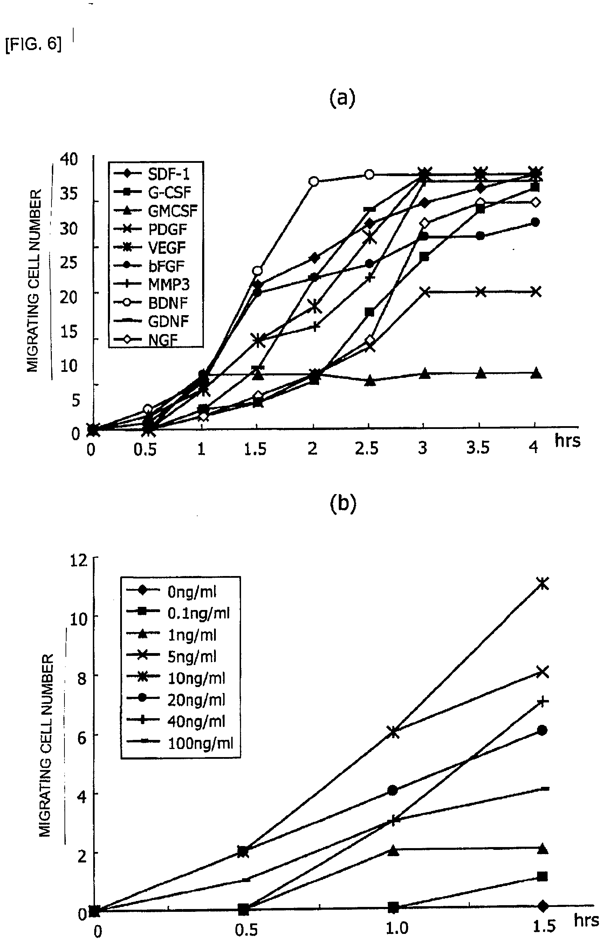

   Fig. 6(a) is a view showing differences in the migration of dog CD105-positive cells depending on the types of cell migration factors, analyzed by TaxiScan, and time course; and
   Fig. 6(b) is a view showing differences in the migration of dog CD105-positive cells depending on various concentrations of the cell migration factor G-CSF, analyzed by TaxiScan.
[Fig. 7]

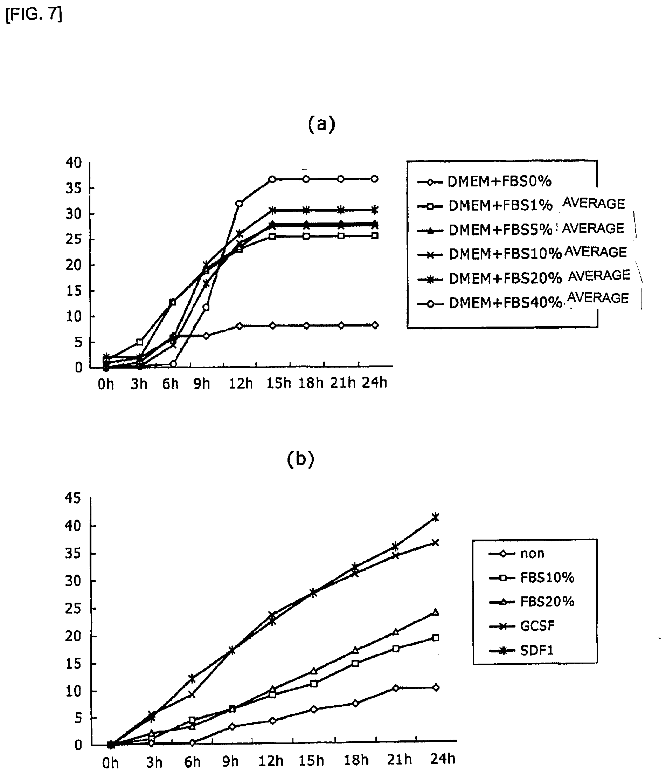

   Fig. 7(a) is a view showing differences in the migration of human dental pulp test cells depending on a change in fetal bovine serum concentrations, analyzed by TaxiScan, and time course; and Fig. 7(b) is a view showing differences in the migration of human dental pulp test cells by the cell migration factors G-CSF and SDF-1 (final concentration: 100 ng/ml) in comparison with fetal bovine serum, analyzed by TaxiScan.
[Fig. 8]

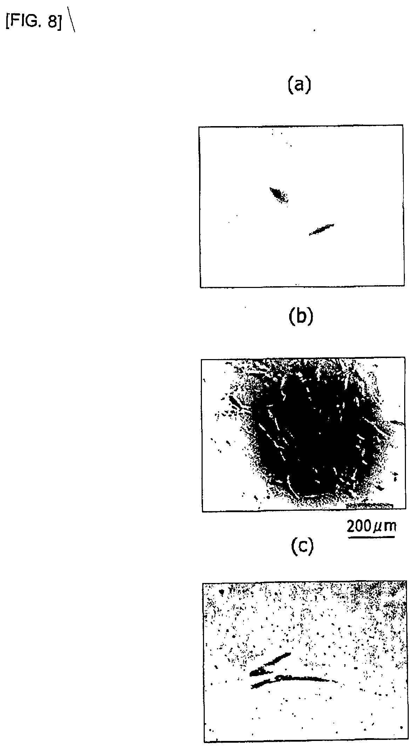

   Fig. 8(a) is a view showing dental pulp stem cells obtained by dispersing fresh dog primary dental pulp cells (1 × 10⁵ cells/250 µl) in an upper portion of a separation membrane by use of the membrane separation culture device according to the present invention (2 × 10⁵ pores/cm², pore size: 3 µm), placing 100 ng/ml G-CSF in DMEM containing 10% dog serum in a lower structure of the separation membrane, then, 6 hours later, replacing the medium with fresh medium, then removing the G-CSF, and then further performing a culture for 1 day; Fig. 8(b) is a view showing dental pulp stem cells obtained by dispersing fresh dog primary dental pulp cells (1 × 10⁵ cells/250 µl) in an upper portion of a separation membrane by use of the membrane separation culture device according to the present invention (2 × 10⁵ pores/cm², pore size: 3 µm), placing 100 ng/ml G-CSF in DMEM containing 10% dog serum in a lower structure of the separation membrane, then, 6 hours later, replacing the medium with fresh medium, then removing the G-CSF, and then further performing a culture for 7 days; and Fig. 8(c) is a view showing dental pulp stem cells obtained by dispersing fresh dog primary dental pulp cells (1 × 10⁵ cells/250 µl) in an upper portion of a separation membrane by use of the membrane separation culture device according to the present invention (2 × 10⁵ pores/cm², pore size: 3 µm), placing 100 ng/ml SDF-1 in DMEM containing 10% dog serum in a lower structure of the separation membrane, then, 6 hours later, replacing the medium with fresh medium, then removing the SDF-1, and then further performing a culture for 1 day.
[Fig. 9]

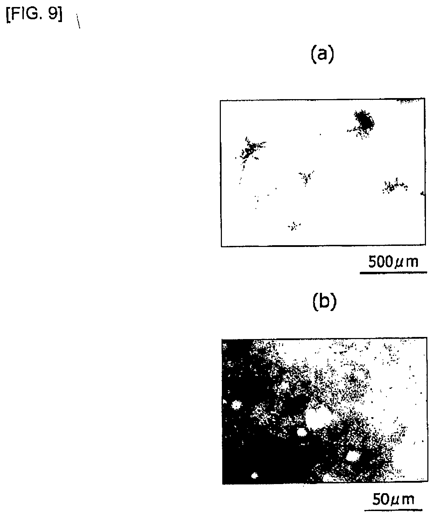

   Fig. 9(a) is a view showing the angiogenesis-inducing ability of the 5th-generation dental pulp stem cells in a test tube, which were separated and cultured using the membrane separation culture device according to the present invention and G-CSF; and Fig. 9(b) is a view showing the neurosphere-forming ability of the 5th-generation dental pulp stem cells in a test tube, which were separated and cultured using the membrane separation culture device according to the present invention and G-CSF.
[Fig. 10]

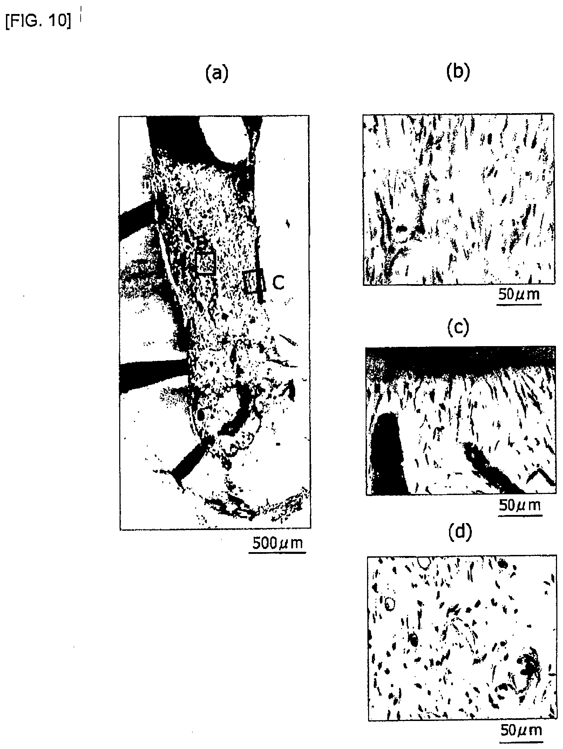

   Fig. 10(a) is a view showing regeneration of dental pulp observed 14 days after transplantation of dental pulp stem cells, which had been separated and cultured using the membrane separation culture device according to the present invention and G-CSF, into the root canal after extirpation of dog pulp; Fig. 10(b) is a high magnification view of the site shown in B of Fig. 10(a); Fig. 10(c) is a high magnification view of the site shown in C of Fig. 10(a), which shows odontoblasts differentiated and aligned along the dentin side wall of the regenerated dental pulp in the root canal; and Fig. 10(d) is a view showing a normal dental pulp of a dog of the same age at the same site.
[Fig. 11]

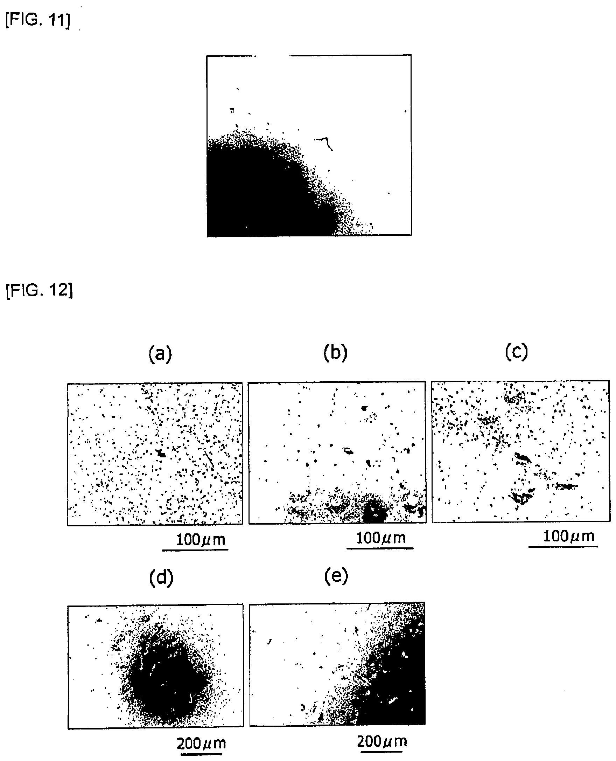

   Fig. 11 is a view showing human dental pulp stem cells obtained by dispersing fresh human primary dental pulp cells (1 × 10⁵ cells/250 µl) in an upper portion of a separation membrane by use of the membrane separation culture device according to the present invention (2 × 10⁵ pores/cm², pore size: 3 µm), placing 100 ng/ml G-CSF in DMEM containing 10% human serum in a lower structure of the separation membrane, then, 6 hours later, replacing the medium with fresh medium, then removing the G-CSF, and then further performing a culture for 8 days.
[Fig. 12]
   Fig. 12(a) is a view showing the separated human dental pulp stem cells obtained by dispersing fresh human primary dental pulp cells (1 × 10⁵ cells/100 µl) in an upper portion of a separation membrane by use of the membrane separation culture device according to the present invention (1 × 10⁵ pores/cm², pore size: 8 µm), placing DMEM containing 10% human serum in a lower structure of the separation membrane, then, 22 hours later, replacing the medium with fresh medium, and then further performing a culture for 3 days; Fig. 12(b) is a view showing human dental pulp stem cells obtained by dispersing fresh human primary dental pulp cells (1 × 10⁵ cells/100 µl) in an upper portion of a separation membrane by use of the same membrane separation culture device as mentioned above, placing 10 ng/ml G-CSF in DMEM containing 10% human serum in a lower structure of the separation membrane, then, 22 hours later, replacing the medium with fresh medium, then removing the G-CSF, and then further performing a culture for 3 days; Fig. 12(c) is a view showing human dental pulp stem cells obtained by dispersing fresh human primary dental pulp cells (1 × 10⁵ cells/100 µl) in an upper portion of a separation membrane by use of the same membrane separation culture device as mentioned above, placing 100 ng/ml G-CSF in DMEM containing 10% human serum in a lower structure of the separation membrane, then, 22 hours later, replacing the medium with fresh medium, then removing the G-CSF, and then further performing a culture for 3 days; Fig. 12(d) is a view showing human dental pulp stem cells observed 7 days after completion of the culture of 10% human serum described in Fig. 12(a) above; and Fig. 12(e) is a view showing human dental pulp stem cells observed 7 days after completion of the culture of 100 ng/ml G-CSF described in Fig. 12(c) above.
[Fig. 13]

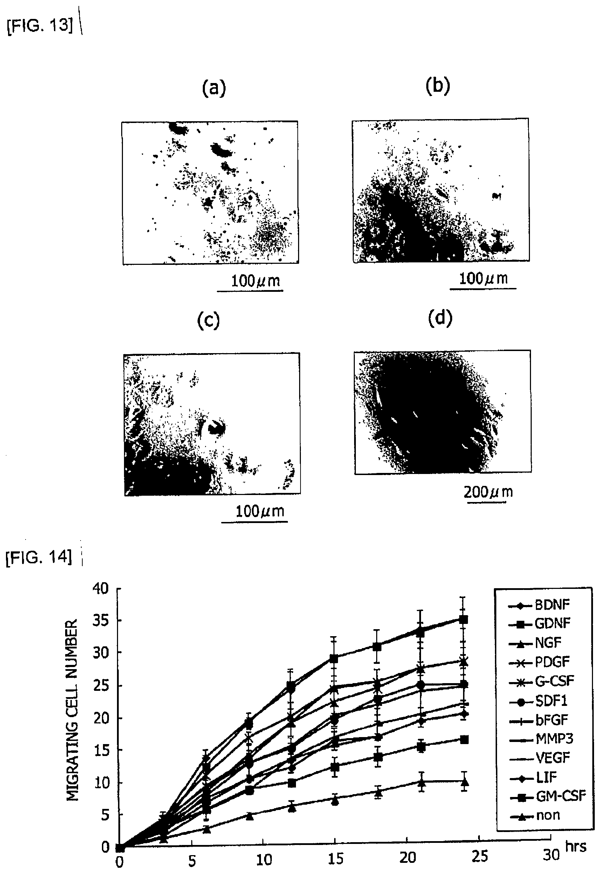

   Fig. 13(a) is a view showing pig dental pulp stem cells obtained by dispersing fresh pig primary dental pulp cells (1 × 10⁵ cells/100 µl) in an upper portion of a separation membrane by use of the membrane separation culture device according to the present invention (1 × 10⁵ pores/cm², pore size: 8 µm), placing 100 ng/ml G-CSF in DMEM containing 10% fetal bovine serum in a lower structure of the separation membrane, then, 22 hours later, replacing the medium with fresh medium, then removing the G-CSF, and then further performing a culture for 3 days; Fig. 13(b) is a view showing pig bone marrow stem cells obtained by dispersing fresh pig primary bone marrow cells, instead of fresh pig primary dental pulp cells, then performing membrane separation, and then performing a culture for 3 days; Fig. 13(c) is a view showing pig adipose stem cells obtained by dispersing fresh pig primary adipose cells, instead of fresh pig primary dental pulp cells described in Fig. 13(a) above, and then performing a culture for 3 days; and Fig. 13(d) is a view showing pig dental pulp stem cells obtained by dispersing the fresh pig primary dental pulp cells described in Fig. 13(a) above, then performing membrane separation, and then performing a culture for 8 days.
[Fig. 14]
   Fig. 14 is a graph showing a comparison regarding the migration ability of unseparated human dental pulp test cells depending on various types of migration factors.
[Fig. 15]

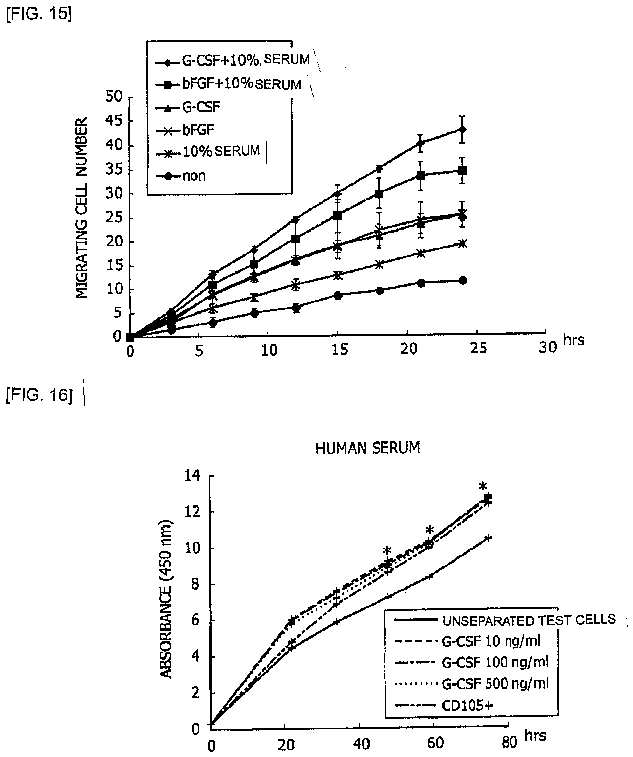

   Fig. 15 is a graph showing a comparison regarding the migration ability of human dental pulp test cells depending on the formulated migration factors and serum.
[Fig. 16]
   Fig. 16 is a graph showing a comparison regarding the cell proliferative ability of membrane-separated dental pulp cells that had been separated using various concentrations of G-CSF, wherein human serum was used.
[Fig. 17]

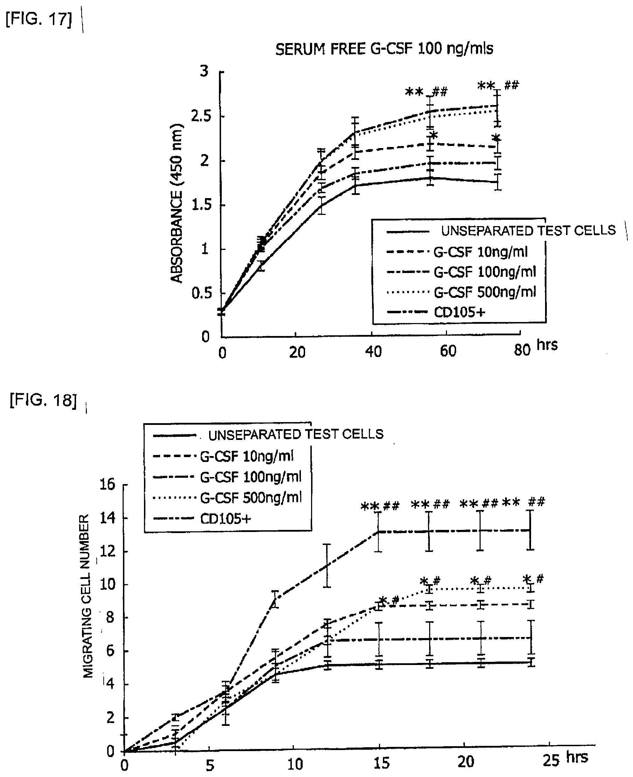

   Fig. 17 is a graph showing a comparison regarding the cell proliferative ability of membrane-separated dental pulp cells that had been separated using various concentrations of G-CSF, wherein 100 ng/ml G-CSF was used.
[Fig. 18]
   Fig. 18 is a graph showing a comparison regarding the cell migration ability of membrane-separated dental pulp cells to G-CSF that had been separated using various concentrations of G-CSF.
[Fig. 19]

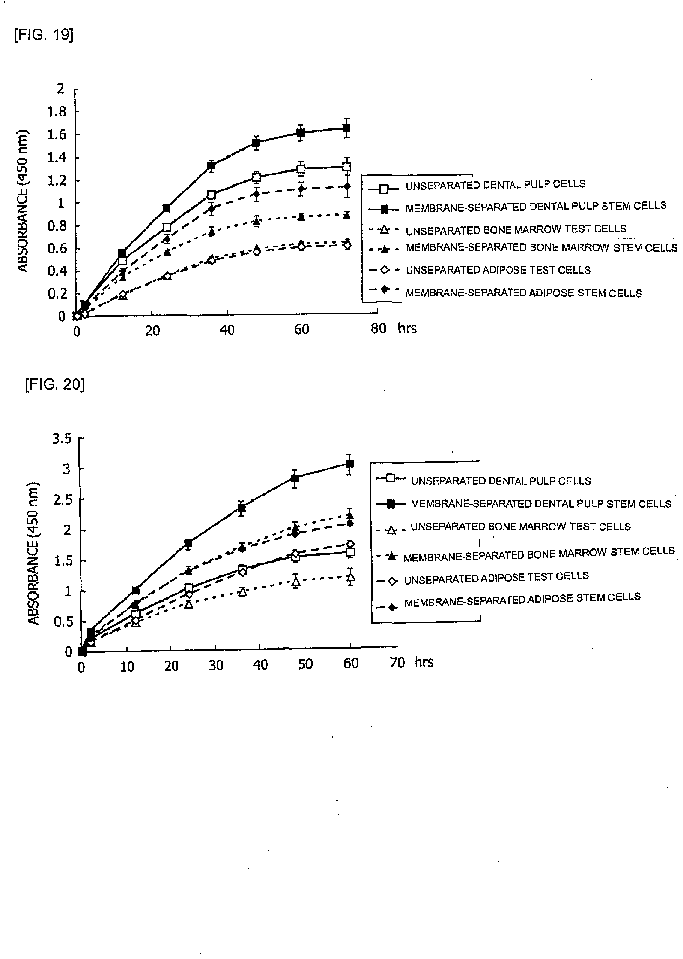

   Fig. 19 is a graph showing a comparison regarding cell proliferative ability to fetal bovine serum, which was made among membrane-separated dental pulp, bone marrow and adipose cells that had been each separated using 100 ng/ml G-CSF, and test cells.
[Fig. 20]
   Fig. 20 is a graph showing a comparison regarding cell proliferative ability to 100 ng/ml G-CSF, which was made among membrane-separated dental pulp, bone marrow and adipose cells that had been each separated using 100 ng/ml G-CSF, and test cells.
[Fig. 21]

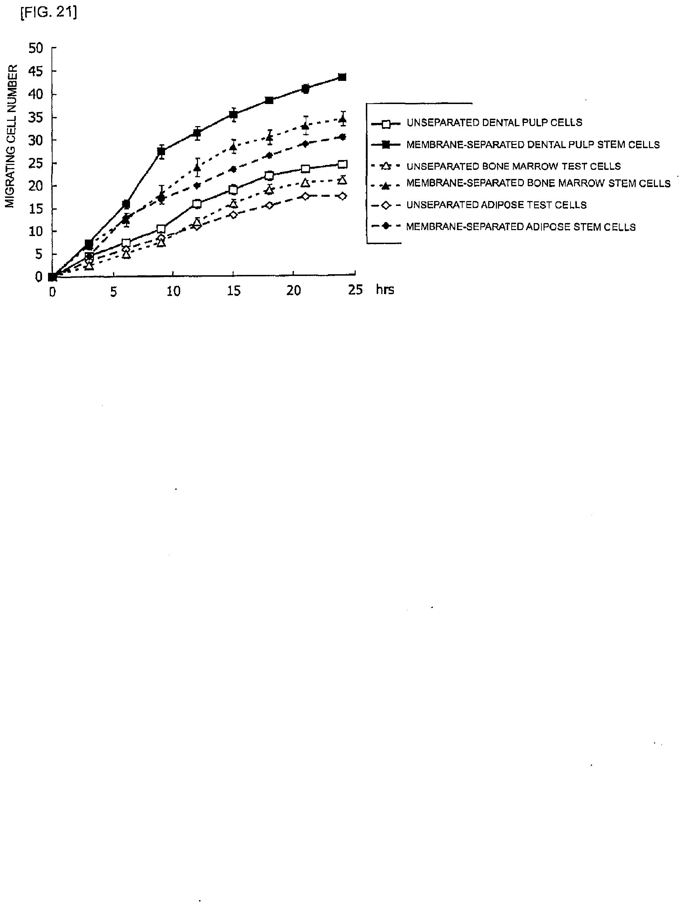

   Fig. 21 is a graph showing a comparison regarding migration ability to G-CSF, which was made among membrane-separated dental pulp, bone marrow and adipose cells that had been each separated using 100 ng/ml G-CSF, and test cells.
[Fig. 22]

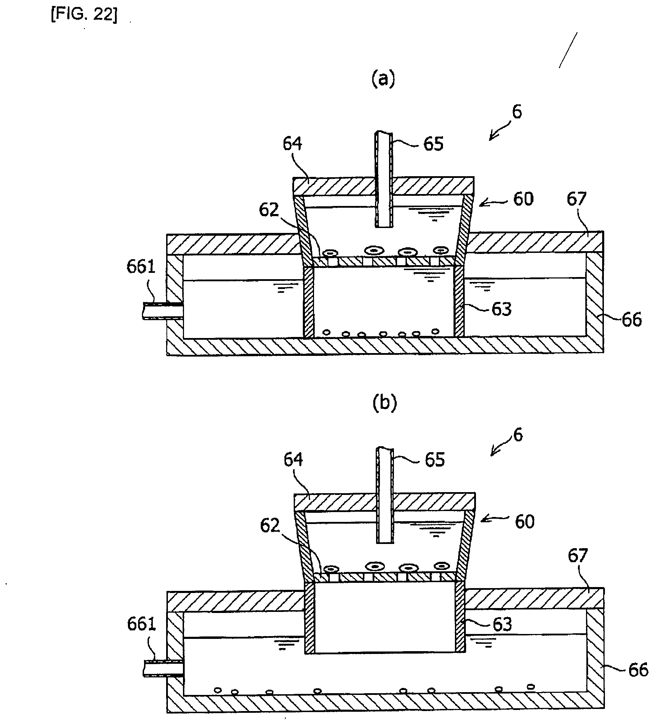

   Fig. 22 is a schematic view showing a configuration of a membrane separation culture device according to a sixth embodiment.
[Fig. 23]

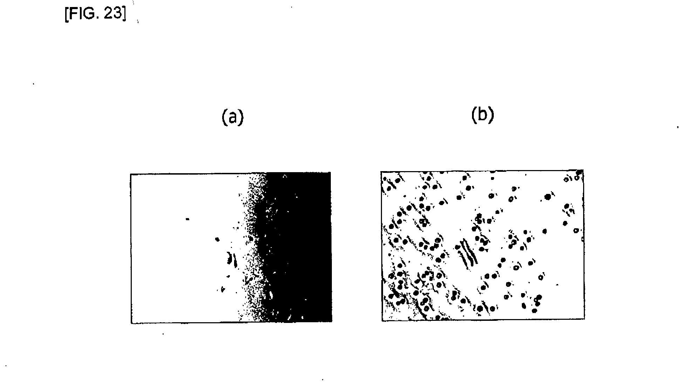

   Fig. 23(a) is a view showing dental pulp stem cells obtained by leaving at rest minced fresh dog dental pulp tissues (2 mg/200 µl) on an upper portion of a membrane by use of the membrane separation culture device according to the present invention (1 × 10⁵ pores/cm², pore size: 8 µm), then placing 100 ng/ml G-CSF in DMEM containing 10% serum in a lower structure of the membrane, and then leaving them for 24 hours; and Fig. 23(b) is a view showing adipose stem cells, 24 hours after being obtained in the same manner as in Fig. 23(a) above by leaving at rest minced fresh dog adipose tissues (2 mg/200 µl) on an upper portion of a membrane, then placing 100 ng/ml G-CSF in DMEM containing 10% serum in a lower structure of the membrane, and then leaving them for 24 hours.

### Description of Embodiments

Hereinafter, the present invention will be described in detail, while referring to the figures.

### (First Embodiment)

A membrane separation culture device 1 according to a first embodiment of the present invention, as shown in Fig. 1, is composed of an upper structure 10 and a lower structure 13. The upper structure 10 is configured to contain a medium 100 containing test cells 200 or test tissues and to retain the test cells 200 or test tissues on a separation membrane 12. On the other hand, the lower structure 13 is configured to contain a medium 300 containing migration factor(s) (not shown in the figure) and receive migrating stem cells.

The upper structure 10 is a vessel consisting of a lateral surface portion 11 and a round-shaped bottom surface portion, wherein the bottom surface portion is formed with the separation membrane 12 having a plurality of pores 121. The type of the upper structure 10 is not particularly limited, as long as it can contain the medium 100 and the test cells 200 or the test tissues therein. In the present specification, the term "test cells" is used to mean cells which have been released from intercellular adhesion in tissues by enzyme treatment and have not yet been separated, or cells which have been subcultured and dispersed. For example, the vessel is preferably capable of containing approximately 100 to 250 µl of the medium 100 and the test cells 200. The term "test tissues" is used herein to mean tissues which have been minced, but have not been digested with enzyme and have not been dispersed.

The separation membrane 12 constituting the bottom surface of the upper structure 10 has a plurality of pores 121 for allowing stem cells to pass therethrough. The pore size is 3 µm to 10 µm, and preferably 5 µm to 8 µm. This is because stem cells are allowed to permeate through the pores. In addition, the pore density is 1 × 10⁵ to 4 × 10⁶ pores/cm². In order to allow stem cells to efficiently pass through the pores, the higher the porosity rate, the better results that can be obtained.

As a material for the separation membrane 12, it is preferable to use a material comprising, as a base material, a hydrophobic polymer such as PET, polycarbonate, polysulfone, polypropylene, polyvinylidene fluoride or polyamide. Moreover, the thickness of the separation membrane 12 is set at preferably 10 to 100 µm, and more preferably 10 to 25 µm. This is because the surface of stem cells is not damaged when the stem cells migrate, and in particular, when the stem cells are allowed to pass through pores.

In order for the separation membrane 12 to have a non-cell-adhesive property, the surface of the separation membrane is preferably coated with a coating agent. In particular, such a coating agent may be applied to an inner surface of the upper structure 10, which is a surface allowed to come into contact with the test cells 200 or test tissues, when the test cells 200 or test tissues are dispersed. Examples of the coating agent that can be used herein include known non-cell-adhesive coating agents, such as an ethyleneoxide/propyleneoxide copolymer (trade name: Pluronic F108, ADEKA CORPORATION), coating agents in which poly2-hydroxyethyl methacrylate is dissolved in 95% ethanol to a concentration of 5 mg/ml (Folkman J & Moscona A, Nature 273: 345-349, 1978, Japanese Patent Application Laid-Open No. 8-9966, etc.), and a branched polyalkylene glycol derivative (WO2009/072590), but the examples are not limited thereto. Any given non-cell-adhesive coating agents can be used. The coating thickness is not particularly limited, as long as it is in a range necessary for imparting a sufficient non-cell-adhesive property to a base material membrane such as PET, polycarbonate or polyvinylidene fluoride. Thus, for example, the thickness of the coating agent is set at preferably 10 to 100 µm, and more preferably 10 to 25 µm, although it depends on the type of the coating agent.

A particularly preferred method for modifying the separation membrane 12 will be further described. In the above-described methods using coating agents, the remaining organic solvent may have adverse effects on cells, such that the pores of the separation membrane may be clogged with the remaining organic solvent, or elution may occur with an aqueous culture medium. Accordingly, the surface of the separation membrane is preferably modified by the following covalent bond method.

That is to say, a base material membrane consisting of a hydrophobic polymer, on which pores with a desired pore diameter have been formed at a high porosity rate, is immersed in a treating aqueous solution comprising a vinyl pyrrolidone polymer, an ethylene glycol polymer and/or a vinyl alcohol polymer, and as necessary, an alcohol, and thereafter, the base material membrane is irradiated with a high-energy beam, so as to perform surface modification, thereby producing a separation membrane.

The polyvinyl pyrrolidone polymer is a polymer selected from the group consisting of polyvinyl pyrrolidone, a vinyl pyrrolidone/vinyl acetate copolymer, a vinyl pyrrolidone/vinyl alcohol copolymer, a vinyl pyrrolidone/styrene copolymer, a vinyl pyrrolidone/dimethyl aminoethyl methacrylate copolymer, and a modified polymer thereof. The ethylene glycol polymer includes those containing an ester group on the side chain thereof. As vinyl alcohol polymers, various types of polymers can be obtained depending on saponification degree. However, the type of such a vinyl alcohol polymer is not limited. These membrane surface-modifying polymers are preferably water-soluble polymers. Thus, polymers having a number average molecular weight of 10,000 to 1,000,000 can be used, for example. However, as long as the polymer is water-soluble, its molecular weight is not limited to the aforementioned molecular weight. The concentration of a polypyrrolidone polymer in the aforementioned treating aqueous solution is preferably 10 to 5000 ppm. If the concentration of the polymer becomes high, pores may be clogged with the treating aqueous solution. Accordingly, the concentration of the polypyrrolidone polymer is more preferably 10 to 2000 ppm.

Moreover, in order to efficiently modify the surface of the base material membrane, when the base material membrane has a water absorption percentage of 2% or less, it is preferable to further add an alcohol to the treating aqueous solution. It is to be noted that the water absorption percentage of the base material membrane is defined as a weight increase percentage obtained by immersing a base material membrane having a thickness of 100 µm or less in water at 23°C for 24 hours and then measuring the weight increased.

If taking into consideration safety when an alcohol remains, the alcohol that is added when the water absorption percentage of the base material membrane is 2% or less is preferably ethanol. The concentration of the alcohol added is preferably 1% by weight or less, and for safety, it is more preferably 0.5% by weight or less, and further preferably 0.1% by weight or less, based on the weight of the treating aqueous solution.

As a high-energy beam, any of UV, an electron beam, and a γ-ray can be used. Among them, an electron beam or a γ-ray is more preferable because these easily enhance a reaction rate. The dose to be applied is preferably 5 to 35 kGy. It is also possible to simultaneously carry out surface modification and sterilization by irradiating, in particular, the entire culture device, with a dose of, for example, approximately 25 kGy, which is considered to be a dose used for sterilization.

The thus obtained separation membrane is useful from the viewpoint of non-cell-adhesive property, and thus, it can be effectively used in the membrane separation device according to the present first embodiment.

As a material for the lateral surface portion 11 of the upper structure 10, an ordinary material that is generally used as a cell culture device can be used, and it may be made of plastic such as polyethylene terephthalate (PET), polystyrene, polypropylene (PP) or polycarbonate.

The dimension of the upper structure 10 is not limited to a specific size. For example, the diameter of the bottom surface portion may be set at 5 to 8 mm. The diameter of an opening portion of the vessel, which is specified with the top edge of the lateral surface portion 11, may be set at 6 to 10 mm, for example. The height from the bottom surface portion 12 to the opening portion, namely, the depth of the vessel may be set at 10 to 15 mm, for example. It is to be noted that these values are given only for illustrative purpose, and that the dimension of the vessel constituting the upper structure 10 can be determined by a person skilled in the art, as appropriate, depending on purposes such as the type of test cells or test tissues, or the type of stem cells to be collected.

Next, the lower structure 13 is a vessel consisting of a bottom surface portion and a lateral surface portion, and it has an opening portion on an upper surface. With regard to a material for the lower structure 13, the same materials can be used for both the lateral surface and the bottom surface thereof. It is preferable to use polystyrene, and glass. This is because these materials impart a cell adhesion property and a cell proliferation property to the lower structure. It is to be noted that it is not always necessary that the bottom surface portion be fixed and integrated with the lateral surface portion in the lower structure, but that it is also possible to form the bottom surface portion of the lower structure with a material different from the material of the lower structure depending on embodiments.

The lower structure 13 can be detachably equipped with the upper structure 10. When the upper structure 10 is equipped into the lower structure 13, the separation membrane 12 of the vessel constituting the upper structure 10 is stored in the lower structure 13. This is because a medium contained in the lower structure 13 is allowed to come into contact with the separation membrane 12 constituting the upper structure 10 in the below-mentioned method for separating stem cells, so that it enables the passage of stem cells through the separation membrane. In the embodiment shown in the figure, the separation membrane 12 is not allowed to come into contact with the bottom surface of the lower structure 13, and a space is formed between the separation membrane 12 and the bottom surface portion of the lower structure 13. This space can be filled with a medium.

In order to fix the positional relationship between the lower structure 13 and the upper structure 10 when the lower structure is equipped with the upper structure, the membrane separation culture device may also have a retention mechanism that is not shown in the figure. The retention mechanism may be established on either one of the upper structure 10 and the lower structure 13, or may also be established on both of the two structures. The retention mechanism may be, for example, a flange or an unguiform member that extends from the top edge or a predetermined position of the lateral surface of the upper structure 10 towards the outside of the opening portion. Such a retention mechanism is allowed to come into contact with the top edge of the lateral surface of the lower structure 13, so that it can retain the upper structure 10 at a predetermined height. Another example of the retention mechanism may be a pedate member, which is established on the upper structure 10 and extends downward from the bottom surface of the upper structure 10. Such a retention mechanism is allowed to come into contact with the bottom surface of the lower structure 13, so that it can retain the upper structure 10 at a predetermined height. At this time, it is also possible to establish a member, which is fitted with the above described pedate member to fix it, also on the bottom surface of the lower structure.

In relation to the dimension of the above described upper structure 10, as an example of the dimension of the lower structure 13, the diameter of the bottom surface portion may be set at 7 to 15 mm, for example. In addition, the diameter of the opening portion of the vessel, which is specified with the top edge of the lateral surface portion of the lower structure 13, may also be set to the aforementioned size. The depth of the lower structure 13 is preferably greater than the depth of the upper structure 10, and it may be set at 11 to 20 mm, for example.

In the present first embodiment, an upper structure comprising a bottom surface and an opening portion each having a round shape, wherein the diameter of the bottom surface is smaller than the diameter of the opening portion, is described as an example. However, the shape of the bottom surface portion 12 is not limited to a circle, and the bottom surface portion can also have an elliptical, square, polygonal or any given shape. Moreover, the relationship between the dimension of the bottom surface portion and the dimension of the opening portion is not limited to that as defined in the present embodiment. The dimension of the bottom surface portion may be identical to the dimension of the opening portion. Furthermore, the upper structure may also be configured to contain a partial surface of a sphere in which the bottom surface is consecutive with the lateral surface, as far as it has a separation membrane having a plurality of pores in at least a portion of the bottom surface thereof, which can retain cells that are dispersed thereon. In the present embodiment, regarding the lower structure 13 as well, a lower structure comprising a bottom surface and an opening portion each having a round shape is given as an example. However, the shapes of the bottom surface and the opening portion are not limited to specific shapes. Further, it is not necessary that the bottom surface can be clearly distinguished from the lateral surface in the lower structure, and the lower structure may also be configured to contain a partial surface of a sphere in which the bottom surface is consecutive with the lateral surface.

The thus-described membrane separation culture device 1 can be used to separate stem cells from the tissues or cells of any given organism including mammals. Examples of the stem cells that can be separated herein include non-human embryonic stem cells, iPS cells, and tissue stem cells. The membrane separation culture device 1 can be used to separate dental pulp stem cells or mesenchymal stem cells from dental pulp cells or mesenchymal cells, in particular, for the purpose of regenerating the dental pulp of mammals including humans. Examples of mesenchymal stem cells include bone marrow stem cells, adipose stem cells, amniotic stem cells, and cord blood stem cells. However, the intended use of the membrane separation culture device 1 according to the present embodiment is not limited thereto.

Specifically, the above-described dental pulp stem cells or other tissue stem cells as targets of separation preferably comprise at least any one of CD105-positive cells, CXCR4-positive cells, SSEA-4-positive cells, FLK-1-positive cells, CD31-negative and CD146-negative cells, CD24-positive cells, CD150-positive cells, CD29-positive cells, CD34-positive cells, CD44-positive cells, CD73-positive cells, CD90-positive cells, FLK-1-positive cells, G-CSFR-positive cells, and SP cells, which are derived from the dental pulp or other tissues (e.g. bone marrow, adipose tissues, amnion, periodontal membrane, synovial membrane, or umbilical cord). The SP cells are preferably any one of CXCR4-positive cells, SSEA-4-positive cells, FLK-1-positive cells, CD31-negative and CD146-negative cells, CD24-positive cells, CD105-positive cells, CD150-positive cells, CD29-positive cells, CD34-positive cells, CD44-positive cells, CD73-positive cells, CD90-positive cells, FLK-1-positive cells, and G-CSFR-positive cells.

Next, the membrane separation culture device 1 according to the present embodiment will be described from the viewpoint of a method for separating stem cells. The method for separating stem cells comprises a step of dispersing test cells 200 or test tissues on a separation membrane 12 of an upper structure 10, a step of pouring a medium 300 containing cell migration factor(s) into a lower structure 13, and a step of allowing the separation membrane 12 to come into contact with the medium 300. By these steps, stem cells are allowed to selectively pass from the upper portion of the separation membrane 12, using the concentration gradient of the cell migration factor(s) placed in the lower structure 13, so that the stem cells can be separated.

In the step of dispersing the test cells 200 or test tissues on the separation membrane 12 of the upper structure 10, the test cells 200, which can be obtained by a known method, for example, according to Nakashima, Archs. Oral Biol. 36, 1991, are dissolved in a medium 100, and the thus-obtained solution is then dispersed on the separation membrane 12 of the upper structure 10. The test cells 200 used as a source for separation of stem cells may be dental pulp cells or mesenchymal cells. The mesenchymal cells include cells derived from bone marrow, adipose tissues, amnion, periodontal membrane, synovial membrane, or umbilical cord. In addition, when non-human embryonic stem cells or iPS cells are separated, the test cells 200 used as a separation source can be a non-human embryo, a non-human blastocyst, or somatic cells on which gene introduction or protein introduction has been performed. When the test tissues are used, the tissues are minced, are immersed in a medium, and are then left at rest on the separation membrane 12 of the upper structure 10.

The test cells are dispersed on the separation membrane at a cell density of 3 × 10² cells to 3 × 10⁴ cells per mm² of the separation membrane. For example, the cell density is preferably 1 × 10² cells/100 µl to 1 × 10⁷ cells/100 µl, and more preferably 1 × 10⁴ cells/100 µl to 1 × 10⁶ cells/100 µl, with respect to a separation membrane with a diameter of 6.5 mm. This is because if the cell density is too low, the cells hardly proliferate, and if the cell density is too high, the cells hardly migrate. The quantities of necessary test cells are different depending on the type of stem cells to be separated. For instance, only very small quantities (e.g. approximately 1 × 10⁵ cells) of test cells that are dental pulp tissues are needed to separate 1 × 10³ dental pulp stem cells. The most preferred density of test cells, in particular, in the case of separating dental pulp stem cells is 3 × 10² to 1.5 × 10³ cells/ mm². On the other hand, the densities of test cells required for separating the same quantities of bone marrow stem cells or adipose stem cells as dental pulp stem cells may be, for example, approximately 3 × 10⁵ cells and 1 × 10⁶ cells, respectively. Moreover, when iPS cells are separated, the quantities of the test cells 200 are different depending on introduction efficiency. The necessary quantities of test cells are already known to those skilled in the art, and thus, can be determined as appropriate. Furthermore, when test tissues are used, the test tissues can be left at rest at a density of 0.1 mg to 1 mg per mm² of the separation membrane.

In the step of pouring the medium 300 containing cell migration factor(s) into the lower structure 13, the cell migration factor(s) are dissolved in the medium, and the obtained solution is then poured into the lower structure 13. The cell migration factor(s) added to a medium that is to be placed in the lower structure 13 are at least any one of SDF-1, G-CSF, bFGF, TGF-β, NGF, PDGF, BDNF, GDNF, EGF, VEGF, MMP3, Slit, GM-CSF, LIF, HGF, and serum. Particularly, from the viewpoint of migration activity and safety in clinical use, G-CSF or bFGF is most preferable. Moreover, the concentration of the cell migration factor(s) is preferably 1 ng/ml to 500 ng/ml. If the concentration is too low, there may be cases in which migration effect cannot be obtained. If the concentration is too high, there is a risk that differentiation of stem cells may occur. In particular, when G-CSF or bFGF is used as such a cell migration factor, the concentration of G-CSF or bFGF is preferably 50 to 150 ng/ml, and particularly preferably around approximately 100 ng/ml, for example, 95 to 105 ng/ml. This is because, using the cell migration factor in such a concentration, the largest quantities of stem cells can be separated, and further, the expression level of the mRNA of an angiogenic factor or a neurotrophic factor is high in the thus separated stem cells.

As a medium, Dulbecco's Modified Eagle Medium, and EBM2 can be used. However, the medium used herein is not limited thereto. Any medium, which can be used for the culture of stem cells, may be used. The amount of the medium can be determined, as appropriate, depending on the volume of the lower structure 13. Serum, as well as cell at least one migration factor, is preferably added to the medium. This is because serum has the effect of promoting cell migration activity. In order to separate human stem cells, human serum is preferably used. Also, fetal bovine serum can be used. Such serum is preferably added in an additive amount of 5 to 20 vol % based on the volume of the medium.

In the step of allowing the separation membrane 12 to come into contact with the medium 300, the upper structure 10 is laminated on the lower structure 13, so that the external side of the separation membrane 12 is allowed to sufficiently come into contact with the medium 300. Thereby, the concentration gradient of the at least one cell migration factor can be carried out. As a result, stem cells contained in the test cells 200 or test tissues are allowed to pass through pores 121, and they migrate towards the lower structure 13, so that the stem cells can be separated. The operation time required after the contact of the separation membrane with the medium is preferably 40 to 50 hours.

In the above explanation, the test cells 200 or the test tissues are placed in the upper structure 10, and the medium containing cell migration factor(s) is placed in the lower structure 13, and thereafter, the upper structure 10 is mounted on the lower structure, so that the separation membrane 12 is allowed to come into contact with the medium. However, the order of performing these three steps is not determined. It may also be possible that the upper structure 10 is first combined with the lower structure 13, and that dispersion and pouring are then carried out on the individual structures. Also, these operations may be carried out substantially simultaneously.

This method of culturing stem cells can be carried out regardless of the shape of a specific vessel. In such a case, the method of culturing stem cells comprises a step of allowing test cells or test tissues to come into contact with a non-cell-adhesive surface of a separation membrane having pores, and a step of allowing a medium containing cell migration factor(s) to come into contact with the other surface of the separation membrane.

Using the membrane separation culture device 1 according to the present embodiment and a method for separating stem cells using the same, stem cells can be separated safely and efficiently.

According to a second embodiment of the invention as shown in figure 2 the present invention relates to a membrane separation culture device comprising a gas exchange system and a medium replacement system. Fig. 2 is a conceptual view showing a membrane separation culture device 2 according to the present embodiment. The membrane separation culture device 2 further comprises a lid structure 24, as well as an upper structure 20 and a lower structure 23.

The basic structures, materials and functions of the upper structure 20 and the lower structure 23 are the same as those described in the first embodiment. In the present embodiment, the lower structure 23 further comprises a medium inlet port 231 and a medium outlet port 232. These ports constitute the medium replacement system. The medium inlet port 231 and the medium outlet port 232 are opening portions that connect the inside of the lower structure 23 with the outside. With such opening portions, cell migration factor(s) and/or a medium containing the separated stem cells can be transferred between the lower structure 23 and the outside. That is, a medium d containing the separated stem cells can be removed through the medium outlet port 232, and a fresh medium c containing cell migration factor(s) can be incorporated through the medium inlet port 231. Therefore, the medium inlet port 231 and the medium outlet port 232 may be connected with a tube that is not shown in the figure. With such a medium replacement mechanism, the present membrane separation culture device is advantageous in that a non-open system (hermetically sealed system) capable of complying with GMP avoids bacterial, viral and mycoplasma infection, and enhances safety and efficiency.

On the other hand, the lid structure 24 is a lid structure that is mounted on the upper structure 20 to cover the opening portion of the upper structure 20 and the opening portion of the lower structure 23. It is preferable that the lid structure adhere tightly to the lower structure 23, or to both the upper structure 20 and the lower structure 23, so that the membrane separation culture device 2 can be hermetically sealed from outside air. For the purpose of hermetically sealing the culture device, the lower structure 23 may be equipped with a rubber or silicone packing at the top edge of the lateral surface thereof.

The lid structure 24 further comprises a gas inlet port 241 and a gas discharge port 242. These ports constitute a gas exchange system. The gas inlet port 241 and the gas discharge port 242 are opening portions that communicate the inside of the lid structure with the outside. The gas inlet port 241 and the gas discharge port 242 may be connected with a tube that is not shown in Figures. For example, inert gas such as CO₂ or N₂ can be supplied into the membrane separation culture device 2 via the gas inlet port 241, and the used gas b such as CO₂ can be removed via the gas discharge port 242.

The lid structure 24 may further comprise a silicone membrane having a plurality of pores having a pore size of 100 nm or less, particularly 1 to 100 nm, and preferably 10 to 100 nm. The silicone membrane can be configured to cover the gas inlet port 241 and the gas discharge port 242 in the lid structure. This is because invasion of mycoplasma from outside is blocked. With such a gas exchange system 24, the present membrane separation culture device is advantageous in that a non-open system (hermetically sealed system) capable of complying with GMP enhances safety, efficiency, and survival rate.

The membrane separation culture device according to the present embodiment may further comprise a temperature control system that is not shown in the figure. The temperature control system is composed of a temperature-measuring device for measuring the temperature inside the hermetically sealed membrane separation culture device 2 and a heater/cooler for heating or cooling the membrane separation culture device 2 from the outside thereof. With such a temperature control system, it becomes possible for the present membrane separation culture device to manage the control of temperature, for example, using a temperature-sensitive medium system for the lower structure 23.

In the present embodiment, the membrane separation culture device 2 comprising both a medium replacement mechanism and the gas exchange system 24 is described. However, the membrane separation culture device of the present embodiment is not limited thereto, and it may comprise either one of them. Using the membrane separation culture device 2 according to the second embodiment, a culture that does not particularly need medium replacement can be carried out more safely by a circulating system, and thus, stem cells preferable for tissue regeneration can be efficiently obtained.

According to a third embodiment of the invention as shown in figure 3, the present invention relates to a membrane separation culture device comprising a lid structure. Fig. 3 is a conceptual view showing the configuration of a membrane separation culture device 3 according to the present embodiment. The membrane separation culture device 3 further comprises a lid structure 34, as well as an upper structure 30 and a lower structure 33.

In the present embodiment, the upper structure 30 is a vessel composed of an axial portion having a round-shaped bottom surface and a circular truncated cone portion having an opening portion. The upper structure 30 is configured such that the diameter of the opening portion is greater than the diameter of the bottom surface. The opening portion of the circular truncated cone portion comprises a retention mechanism 35 consisting of a flange that extends outside.

The lower structure 33 shown in the figure is a cylindrical member comprising a round-shaped bottom surface and a round-shaped opening portion, wherein they have the same diameter. The lower structure comprises a groove close to the opening portion, and retains a hermetic sealing elastic body 331 in the groove. The groove may be either a single groove or a double groove. The material of the hermetic sealing elastic body used herein is desirably a synthetic rubber having a clear composition, such as a silicone rubber.
Moreover, around the center of the cylinder and between the groove in which the elastic body 331 is established and the bottom surface, the lower structure 33 also comprises a retention mechanism 335 consisting of a flange extending from the inner wall surface of the cylinder towards the inside. The retention mechanism 335 engages with the retention mechanism 35 of the upper structure 30, so as to retain the upper structure 30 at a predetermined position in the lower structure 33.

The basic structures, materials and functions of the upper structure 30 and the lower structure 33, other than the above descriptions, are the same as those described in the first embodiment.

The lid structure 34 is a member capable of covering or hermetically sealing the upper structure 30 and the lower the structure 33. The lid structure 34 may cover the opening portions of the upper structure 30 and the lower structure 33 from the above of the upper structure 30. The material of the lid structure is the same as that of the lower structure described in the first embodiment. It is desirable that the lid structure 34 further comprise a gas exchange mechanism (not shown in the figure). This gas exchange mechanism may be a plurality of pores having a pore size of 100 nm or less, preferably 1 to 100 nm, and particularly preferably 10 to 100 nm, which are established on the entire surface or a portion of the lid structure 34. As another example of the gas exchange mechanism, a polymer membrane for passing gas, such as a polytetrafluoride ethylene (PTFE) laminated membrane used for gas line filters, may be established in at least a portion of the lid structure 34. The lid structure 34 may further comprise a gas inlet port and a gas discharge port, as described in the second embodiment.

The lid structure 34 is combined with the lower structure 33, so that it can be tightly adhered to the elastic body 331 retained in the lower structure 33. By such a configuration, hermetic sealing can be carried out simply. The present membrane separation culture device further comprises a gas exchange function (not shown in the figure), and as a result, it provides a structure that gives no pressure change to stem cells.

With such a lid structure 34 and a lower structure 33, the membrane separation culture device according to the present embodiment is advantageous in that a risk of contamination can be reduced in the case of covering the lower structure with the lid structure, and in that contamination such as mycoplasma can be avoided and a pressure change due to a temperature change can also be avoided in the case of hermetically sealing the lower structure with the lid structure.

According to a fourth embodiment of the present invention as shown in figure 4, the present invention relates to a membrane separation culture device comprising a plurality of upper structures. Fig. 4 is a conceptual view showing the configuration of a membrane separation culture device 4 according to the present embodiment. The membrane separation culture device 4 comprises a plurality of upper structures 40, a frame body 45 for holding the plurality of the upper structures 40, and a lower structure 43 constituted with a vessel for collectively retaining a fluid in which the separation membranes of the plurality of the upper structures 40 are immersed.

In the present embodiment, the structure of each upper structure 40 may be the same as that in the third embodiment. The plurality of the upper structures 40 may comprise separation membranes each having a different pore size and/or a different pore density, or all of the separation membranes may have the same pore size and/or the same pore density.

The frame body 45 is a structure that is to be contained in the lower structure 43, and it contains the plurality of the upper structures 40 in individual holes 451. Specifically, the frame body 45 is a member in which upper and lower parts are opened, wherein the frame body 45 comprises a plurality of holes 451 established on a plate-like member that is retained at a constant height by a retention mechanism 453. A grid-like partition 452 divides the upper region of the plate-like member to form partitions, and a single hole 451 is present in one partition. The material of the frame body 45 may be the same as that of the lower structure described in the first embodiment. In addition, the retention mechanism 453 is a pedate member extending from the periphery of the plate-like member to the lower portion. The retention mechanism 453 is formed like an outer frame only around the periphery of the plate-like member, and a slit is formed on a portion corresponding to the partition 452 on the upper surface of the plate-like member. Fig. 4 shows the structure of the frame body 45 having 24 holes. However, the number of holes formed on the frame body 45 may be either 2 or 96, and the number of holes is not limited. Moreover, the disposition of holes established on the plate-like member of the frame body 24 is not limited to the embodiment shown in the figure. Furthermore, the retention mechanism 453 in the present embodiment is a pedate member extending from the plate-like member downwards. A flange extending from the inner wall of the lower structure to the inside may be established, so that the retention mechanism 453 may be moored at the flange.

The upper structure 40 can be detachably inserted into each hole 451 on the frame body 45. The hole 451 holds the upper structure 40, such that when the upper structure 40 is inserted, the separation membrane constituting the bottom surface of the upper structure 40 can be positioned between the hole 451 and the inner wall of the bottom surface of the lower structure 43. That is, the hole 451 is formed, such that the diameter of the hole 451 it becomes greater than the diameter of the bottom surface of the upper structure 40 and also becomes smaller than the diameter of the opening portion.

The lower structure 43 is a vessel for detachably containing the frame body 45. A single groove is established on the lower structure 43. In the groove, the elastic body 431 described in the third embodiment is established. The elastic body 431 is configured to be hermetically attached to the after-mentioned lid structure 44 so as to hermetically close the inside of the membrane separation culture device 4. Other structures, materials, and functions of the lower structure 43 are the same as those described in the first embodiment. The lower structure 43 may further comprise a medium inlet port and a medium outlet port (not shown in the figure). The lower structure shown in the figure has a square bottom surface. However, the shape of the lower structure is not limited to a square shape, and it may be circular or elliptical, for example.

The lid structure 44 covers an opening portion between the plurality of the upper structures 40 and the lower structure 43 from the above of the plurality of the upper structures 40. The basic structure, material, and function of the lid structure 44 are the same as those described in the third embodiment. The lid structure 44 may comprise a gas exchange mechanism (not shown in the figure) as described in the third embodiment, or may further comprise a gas inlet port and a gas discharge port, as well as the gas exchange mechanism.

By comprising such a frame body 42 and a lower structure 43, the present membrane separation culture device is advantageous in that large quantities of tissues or cells can be used as analytes and they can be collected by a single lower structure, when the number of stem cells of interest is small, as in the case of iPS cells.

According to a fifth embodiment of the present invention as shown in figure 5, the present invention relates to a membrane separation culture device comprising a plurality of upper structures and a lower structure composed of a plurality of vessels. Fig. 5 is a conceptual view showing the configuration of a membrane separation culture device 5 according to the present embodiment.

The membrane separation culture device 5 comprises a plurality of upper structures 50, a frame body 55, and a lower structure 53 composed of a plurality of vessels each retaining a fluid in which a separation membrane of each upper structure 50 is immersed.

The basic structure and function of the upper structure 50 are the same as those described in the fourth embodiment. In the present embodiment as well, the plurality of the upper structures 50 may comprise separation membranes each having a different pore size and/or a different pore density, or all of the separation membranes may have the same pore size and/or the same pore density.

The basic structure and function of the frame body 55 are the same as those described in the fourth embodiment. In the present embodiment, a retention mechanism 553 as a lower portion of the frame body 55 is equipped with a plurality of slits in order to avoid interference with partitions 532 of the lower structure 53.

On the other hand, the lower structure 53 according to the present embodiment is composed of a plurality of vessels each corresponding to the plurality of the upper structures. Specifically, the main body of the lower structure consists of a plurality of vessels composed of divisions formed by dividing with grid-like partitions 532. The grid-like partitions 532 may be formed by being integrated with the lower structure 53, or may be a member detachable from the lower structure 53. The partitions 532 are fixed on the lower structure to such an extent that substances cannot move between individual vessels formed with such partitions 532 upon use.

The frame body 55 can be mounted on the lower structure 53, and thus, the lower structure 53 can contain the frame body 55. When the frame body 55 can be mounted on the lower structure 53, individual holes 551 of the frame body 52 each correspond to a plurality of vessels of the lower structure 53. In addition, partitions 552 of the frame body 55 are overlapped with the partitions 532 of the lower structure 53. Moreover, each of the plurality of upper structures 50 can be mounted on each hole 551 of the frame body 52. At this time, a combination of one vessel of the lower structure 53 with one upper structure 50 functions as an independent membrane separation culture device. Therefore, each of the vessels divided with the partitions 532 can retain a fluid in which the separation membrane of the upper structure 50 is immersed. For example, different types of fluids containing different migration factor and/or media are placed in different vessels, so as to carry out membrane separation.

The membrane separation culture device 5 according to the present embodiment may also comprise a lid structure that is not shown in the figure. The lid structure may comprise the gas exchange mechanism (not shown in the figure) described in the third embodiment, or may further comprise a gas inlet port and a gas discharge port, as well as the gas exchange mechanism. The lower structure 53 shown in the figure does not have a groove used for hermetically sealing, and it can be used in combination with a covering lid structure.

Using the membrane separation culture device 5 according to the fifth embodiment, in order to separate stem cells that have not yet been separated so far, a plurality of upper structures having different pore sizes are prepared, and a plurality of media containing various cell migration factor(s) are prepared and combined with the upper structures, so that separation conditions can be advantageously screened at one time.

According to a sixth embodiment of the present invention as shown in figure 22, the present invention relates to a closed system membrane separation culture device capable of carrying out subculture. Fig. 22 is a conceptual view showing the configuration of a membrane separation culture device 6 according to the present embodiment.

The membrane separation culture device 6 is essentially composed of a dish 66, as well as an upper structure 60 and a lower structure 63. The basic structure, material, and function of the upper structure 60 are the same as those described in the first embodiment. A lid structure 64 for covering the opening portion of the upper structure 60 from the above of the upper structure 60 is established at the opening portion of the upper structure 60. The lid structure 64 comprises an introduction port 65. The introduction port 65 is a tube preferably made of silicone, which is established by penetrating through the lid structure 64, and is used to communicate the inside of a vessel constituted with the upper structure 60 with the outside. The introduction port 65 is mainly used to insert minced dental pulp tissues or dental pulp test cells into the membrane separation culture device 6 in a closed system, without contamination of the tissues or cells from the outside. Using the introduction port 65, it is also possible to replace a medium with another one in a closed system. The structure, material, and function of a membrane 62 comprised in the upper structure 60 are the same as those described in the first embodiment. On the other hand, the lower structure 63 according to the present embodiment does not have a bottom surface portion integrated and fixed with a lateral surface portion thereof and is composed only of the lateral surface portion. Other than this, the lower structure 63 has the same configuration as that in the first embodiment.

The dish 66 is a vessel composed of a bottom surface portion and a lateral surface portion, and is capable of cell culture. A recovery port 661 for recovering a medium is established on the dish 66, and thereby, medium replacement, the recovery of a culture supernatant, and the recovery of cells can be carried out in a closed system without contamination from the outside. A surface treatment layer that is not shown in the figure is established on the bottom surface portion of the dish 66, which is the inner surface of the vessel. It is preferable that the surface treatment layer has properties excellent in cell adhesion and amplification, be reacted by heat or light, or by both of them, and be degradable. According to one aspect of the disclosure of the present invention, the surface treatment layer can be designed such that it is reacted by irradiation of light with a specific wavelength, and that as a result, a substance constituting the surface treatment layer is decomposed. As an example, poly(N-isopropylacrylamide) is graft polymerized onto the bottom surface portion of the dish 66, which is the inner surface of the vessel, so as to form a surface treatment layer. This surface treatment layer retains hydrophobicity at 37°C, but when the temperature is decreased to approximately 30°C, it is subjected to phase change and is thereby hydrophilized. Thus, it becomes possible to remove cells adhered to the surface of the layer. According to another aspect of the disclosure of the present invention, the surface treatment layer can be designed such that it is reacted by a specific temperature change, and that as a result, a substance constituting the surface treatment layer is decomposed. As an example, a surface treatment layer comprising collagen can be formed. In this case, the surface treatment layer is decomposed by increasing the temperature to a collagen denaturation temperature, and as a result, it becomes possible to remove cells adhering to the surface of the layer.

The dish 66 further comprises, at the upper portion thereof, a lid structure 67 for covering the opening portion of the dish 66 from the above. The lid structure 67 is configured to hermetically seal the inside of the dish 66, and to maintain a hermetically sealed state even if a laminated body of the upper structure 60 and the lower structure 63 moves in the vertical direction.

In the present embodiment, the lower structure 63 is established movably in the vertical direction in the dish 66 and is used. Fig. 22(a) is a conceptual view showing the disposition of individual components when membrane separation is carried out. At this time, the membrane 62, the lateral surface portion of the lower structure 63 and the bottom surface portion of the dish 66 form a closed space, and cells migrating from the upper structure 60 are retained in the space. The diameter of the dish 66 is preferably about 7 to 10 times larger than the diameter of the lower structure 63. It is to be noted that the scale used in Fig. 6 is changed in order to clearly display each member.

It is possible that the lower structure 63 be moved upward in the vertical direction and is then fixed. Fig. 22(b) is a conceptual view showing the membrane separation culture device 6 when the lower structure 63 is moved upward in the vertical direction for medium replacement or the recovery of stem cells. In this case as well, the lid structure 67 can hermetically seal the dish 66.

Next, the membrane separation culture device 6 according to the sixth embodiment will be described from the viewpoint of a closed system culture method. Such a closed system culture method comprises a step of carrying out membrane separation, a step of allowing stem cells to proliferate, a step of subculturing the step cells, and a step of amplifying the step cells and recovering them. In the step of carrying out membrane separation, stem cells are separated by a membrane according to the method described in the first embodiment. In this step, cells that have migrated from the upper structure 60 to the lower structure 63 adhere to the bottom surface of the dish 66 enclosed with the side wall portion of the lower structure 63. Subsequently, in the step of allowing stem cells to proliferate, a laminated body of the upper structure 60 and the lower structure is moved upward in the vertical direction, and the medium is then replaced with a cell growth medium such as DMEM containing 10% serum through the recovery port 661 to remove migration factors, and the laminated body of the upper structure 60 and the lower structure is then moved downward in the vertical direction, so that it is allowed to come into contact with the bottom surface portion of the dish 66 and is fixed thereon, thereby allowing stem cells to proliferate. Thereafter, the step of subculturing the stem cells is carried out. During this step, the laminated body of the upper structure 60 and the lower structure is moved upward in the vertical direction and is then fixed. Then, light or heat is applied onto the surface treatment layer, or the temperature is decreased, so that the surface layer is decomposed and the proliferating stem cells are removed therefrom. At this time, since the side wall portion of the lower structure 63 is not allowed to come into contact with the bottom surface portion of the dish 66, the stem cells are diffused over the entire dish 66. In the step of amplifying the stem cells and recovering them, the subcultured stem cells can be recovered through the medium recovery port 661. During this step, a centrifuge tube is connected with the recovery port 661, and centrifugation is then performed in a closed system, so as to recover cells or a cell supernatant. Moreover, in each step, medium replacement can be carried out through the medium recovery port 661.

In the present embodiment, by establishing a surface treatment layer on the dish 66, it becomes unnecessary for the removal of cells to use an enzyme that has conventionally been used to remove cells from a culture vessel, such as trypsin. Since the obtained cell culture supernatant and cells do not contain enzyme, they can be directly used in transplantation without centrifugation and washing.

According to a seventh embodiment, the present invention relates to a membrane separation culture kit according to claims 3-5. The membrane separation culture kit comprises a membrane separation culture device and cell migration factor(s). As a membrane separation culture device, any of the membrane separation culture devices 1 to 6 according to the above described first to sixth embodiments, or modified forms thereof can be used.

The cell migration factor is at least one of SDF-1, G-CSF, bFGF, TGF-β, NGF, PDGF, BDNF, GDNF, EGF, VEGF, MMP3, Slit, GM-CSF, LIF, HGF, and serum. In addition, the concentration of the cell migration factor is preferably 1 ng/ml to 500 ng/ml. This is for efficient migration of stem cells. That is to say, if the concentration of the migration factor is too low, the migration effect may not be obtained. In contrast, if the concentration is too high, the cells may be differentiated. The cell migration factor may be added to a medium and may be then used. Accordingly, the kit according to the present embodiment may also comprise a medium. As a medium, Dulbecco's modified Eagle's medium, or EBM2 can be used, but examples of the medium used herein are not limited thereto.

Particularly preferably, the kit according to the present embodiment comprises, as a kit-constituting member, a cell migration factor that is G-CSF or bFGF, preferably in a concentration of 50 to 150 ng/ml. Moreover, in addition to the cell migration factor, the present kit may also comprise, as a kit-constituting member, another component to be added to a medium that is human autoserum or fetal bovine serum. Such a serum is configured to be added in a concentration of 5 vol% to 20 vol% based on the total volume of the medium. The kit can be produced and used in accordance with the explanations regarding the devices and methods of the first to fifth embodiments.

According to the membrane separation culture kit of the present embodiment, using a membrane separation culture device and cell migration factor(s) used for separation, the method for separating stem cells that is specifically described can be promptly carried out.

Since the remaining organic solvent may have adverse effects on cells, such that the pores of the separation membrane may be clogged with the remaining organic solvent, or elution may occur with an aqueous culture medium, it is preferable that membrane surface modification be carried out on the separation membrane according to a covalent bond method in the present invention. Specifically, a base material membrane, on which pores each having a desired pore diameter have been formed at a high porosity rate, is immersed in a treating aqueous solution, to which a vinyl pyrrolidone polymer, and/or ethylene glycol polymer, and/or vinyl alcohol polymer, and optionally, an alcohol have been added. Thereafter, the base material membrane is irradiated with a high-energy beam, so as to the surface of the base material membrane can be modified.

The polymer used as a base material membrane of the separation membrane used in the present invention is a hydrophobic polymer. The term "hydrophobic polymer" is used in the present invention to indicate a polymer whose solubility in 100 g of water at 20°C is less than 0.001 g. The hydrophobic polymer is specifically selected from the group consisting of a sulfone polymer an amide polymer, a carbonate polymer, an ester polymer, a urethane polymer, an olefin polymer, and an imide polymer, but examples of the hydrophobic polymer are not limited thereto. Surface modification conditions are changed based on the water absorption percentage of such a hydrophobic polymer constituting the base material membrane, so that a protein or cell adhesion-suppressing property can be more efficiently imparted to the separation membrane.

For cell separation, a membrane having pores with a diameter of 3 to 10 µm is used. Thus, a pore diameter may be selected depending on intended use. **In** particular, the separation membrane used in the present invention can be preferably used for separation in which cell chemotaxis is utilized. **In** this case, a pore diameter of 3 to 8 µm is easily used.

The polymer constituting such a base material membrane generally has strong hydrophobicity, and it is likely that many proteins or cells adhere thereto. **In** particular, since activated proteins or platelets, or adherent cells easily adhere onto the surface of the membrane, it has been concluded that a certain level of surface modification needs to be uniformly carried out, namely, that the covalent bond of the hydrophobic polymer with a hydrophilic polymer is necessary.

The vinyl pyrrolidone polymer is a polymer selected from the group consisting of polyvinyl pyrrolidone, a vinyl pyrrolidone/vinyl acetate copolymer, a vinyl pyrrolidone/vinyl alcohol copolymer, a vinyl pyrrolidone/styrene copolymer, a vinyl pyrrolidone/dimethyl aminoethyl methacrylate copolymer, and a modified polymer thereof. The ethylene glycol polymer includes those containing an ester group on the side chain thereof. As vinyl alcohol polymers, various types of polymers can be obtained depending on saponification degree. However, the type of such a vinyl alcohol polymer is not limited. In the present invention, these polymers are referred to as hydrophilic polymers. These hydrophilic polymers used for membrane surface modification are preferably water-soluble. Thus, polymers having a number average molecular weight of 10,000 to 1,000,000 can be used, for example. However, as long as the polymer is water-soluble, its molecular weight is not limited to the aforementioned molecular weight.

The term "water-soluble polymer" is used in the present invention to mean a polymer for which solubility in 100 g of water at 20°C is 1 g or more, and preferably 10 g or more. From the viewpoint of suppression of adhesion of proteins, platelets, and adherent cells, the separation membrane used in the present invention preferably contains such a water-soluble polymer. An appropriate balance between hydrophilicity and hydrophobicity on the surface has been considered important for suppression of adhesion of proteins or platelets. As a matter of fact, it was found that, when a water-soluble polymer having stronger hydrophilicity is present, the effect of suppressing the adhesion of proteins, platelets, and adherent cells is further improved.

The amount of a water-soluble polymer contained in the separation membrane is preferably 1.5% or more, and more preferably 5% or more, based on the total weight of the separation membrane. In addition, since the effect is not changed even if the separation membrane contains an excessively large amount of water-soluble polymer , the upper limit of the water-soluble polymer is preferably 40% or less, and more preferably 35% or less, based on the total weight of the separation membrane.

Furthermore, if the hydrophilic polymer is a copolymer having a water-soluble unit and an ester group unit, it has an appropriate balance between hydrophilicity and hydrophobicity in a single molecule thereof. Thus, the hydrophilic polymer is preferably such a copolymer. As such a copolymer, a block copolymer, an alternating copolymer, and a random copolymer are preferably used, rather than a graft copolymer. This is because, in the case of a graft copolymer, since a unit portion grafted to a main chain is allowed to often come into contact with a protein, the properties of a graft chain portion become greater than the properties of a copolymer. Further, an alternating copolymer and a random copolymer are more preferable than a block copolymer. This is because, in the case of a block copolymer, the properties of individual units are clearly different from one another. In terms of a balance between hydrophilicity and hydrophobicity in a single molecule, a copolymer having at least one selected from a random copolymer and an alternating copolymer is preferably used. The molar ratio of an ester group unit in an ester group-containing polymer is preferably 0.3 or more and 0.7 or less. If the molar ratio of the ester group unit is less than 0.3, the adhesion-suppressing effect of the ester group is reduced. On the other hand, if the molar ratio of the ester group unit exceeds 0.7, the effect of the water-soluble unit is reduced.

The amount of a hydrophilic polymer serving as a surface-modifying polymer on the surface of a separation membrane can be measured, for example, by elementary analysis, nuclear magnetic resonance (NMR) measurement, or a combination of ESCA and attenuated total reflection method (hereinafter also referred to as ATR). This is because ESCA is used to measure a depth of about 10 nm from the surface, whereas ATR is used for surface measurement that is the measurement of the composition of a depth of several µm. Taking a polysulfone separation membrane as an example, when the ratio of the amount of a hydrophilic polymer to the amount of a polysulfone unit in any given site in the membrane is defined as a unit amount ratio, if the value of the unit amount ratio obtained by ESCA is 30% or more greater than the value obtained by ATR, it can be determined that the amount of an ester group-containing polymer on the membrane surface of the present invention is 30% or more greater than the amount inside the membrane. It is to be noted that the value of each measurement is indicated as a mean value of three sites.

Next, a method for modifying the surface of a molded body will be described. The method for modifying the surface of a molded body comprises: an immersion step of immersing a molded body consisting of a hydrophobic polymer in a treating aqueous solution containing one or more hydrophilic polymers selected from a vinyl pyrrolidone polymer, a polyethylene glycol polymer and a vinyl alcohol polymer at a concentration of 10 to 2000 ppm, and further optionally containing a 0.01 wt % to 0.2 wt % alcohol; and a modification step of irradiating the molded body obtained by the immersion step with a high-energy beam to modify the surface of the molded body to have a protein adhesion-suppressing property and a cell adhesion-suppressing property. When the molded body is a specific base material membrane, such a method for modifying the surface of a molded body can also be referred to as a method for producing a separation membrane, as described above. This method is preferable because it can be easily carried out with a small amount of treating solution.

The molded body consisting of a hydrophobic polymer as defined herein is not limited to a membrane, and it may also be a molded body having a specific shape. When a membrane is used as such a molded body, it may be a base material membrane described in the configuration of the above described separation membrane. In such a case, the present method for modifying the surface of a molded body may be equal to a method for producing a separation membrane.

The treating aqueous solution is an aqueous solution, in which a molded body, consisting of a hydrophobic polymer is to be immersed. The treating aqueous solution contains one or more hydrophilic polymers selected from a vinyl pyrrolidone polymer, a polyethylene glycol polymer and a vinyl alcohol polymer, at a total concentration of preferably 10 to 5,000 ppm, and more preferably 10 to 2,000 ppm. It is to be noted that a specific concentration may be different depending on the type of a hydrophilic polymer. If the concentration of the hydrophilic polymer solution is low, there is a case in which a molded body consisting of a hydrophobic polymer may not be sufficiently coated with the solution. On the other hand, if the concentration is too high, when the molded body is a membrane for example, pores may be clogged, the amount of an elution product may be increased, or the performance of a separation membrane may be reduced in many cases. As described later, surface modification can be more efficiently carried out by addition of an alcohol. **In** such a case, the aforementioned concentration can be set lower than that as mentioned herein.

Moreover, in order to efficiently carry out surface modification, it is preferable to change the composition of the treating aqueous solution depending on the water absorption percentage of the material for the molded body consisting of a hydrophobic polymer. In the present invention, the water absorption percentage of the molded body consisting of a hydrophobic polymer can be obtained by washing with purified water, a membrane having a thickness of 30 to 100 µm that is a material for the molded body, then drying the membrane, and then immersing the dried membrane in water at 23°C for 24 hours, followed by measuring an increased percentage in weight. This weight increase percentage is defined as a water absorption percentage. On the other hand, when the molded body is a separation membrane having a thickness of 200 µm or less, it is directly washed with purified water, is then dried, and is then immersed in water at 23°C for 24 hours, and thereafter, the water absorption percentage can be calculated from a weight increase percentage during this operation.

When the water absorption percentage of a molded body consisting of a hydrophobic polymer is 2% or less, it is preferable to further add an alcohol to a treating aqueous solution used. This is because the surface of the molded body consisting of a hydrophobic polymer can be uniformly coated with the treating aqueous solution as a result of the coexistence of the alcohol. Taking into consideration safety in a case in which the treating aqueous solution remains, the alcohol added is preferably ethanol, but examples of the alcohol added are not limited thereto. It is also possible to use a polyhydric alcohol such as glycerin. The concentration of the alcohol added is preferably 1% or less based on the total weight of the treating aqueous solution. For the sake of safety, it is more preferably 0.5% or less, and further preferably 0.1% or less, based on the total weight of the treating aqueous solution. Since surface modification can be efficiently carried out by previously enhancing an adsorption percentage with an alcohol, the same level of surface modification can be realized even with the use of a lower concentration of hydrophilic polymer. That is to say, the amount of a hydrophilic polymer used can be reduced, and it is effective for cost reduction during production.

On the other hand, when the water absorption percentage of a molded body consisting of a hydrophobic polymer exceeds 2%, it is not necessary to add an alcohol to a treating aqueous solution.

In the immersion step, the entire molded body may be immersed in the treating aqueous solution, or only a portion of the molded body that is to be subjected to surface modification may be immersed in the treating aqueous solution.

The high-energy beam used in the modification step may be UV, an electron beam, a γ-ray, or an X-ray. Of these, an electron beam or a γ-ray is more preferable because it easily enhances a reaction rate. In addition, in terms of a small amount of residual toxicity or simplicity, a γ-ray or an electron beam is preferably used. The applied dose is preferably 5 to 35 kGy. In particular, by irradiating the culture device as a whole with, for example, a dose of approximately 25 kGy that is considered to be a sterilization dose, surface modification and sterilization can be simultaneously carried out. However, if the applied dose is 100 kGy or more, productivity is reduced, and decomposition of a polymer occurs. Thus, application of an excessively high dose is not preferable.

It has been known that, when the surface of the membrane is irradiated with a high-energy beam, if oxygen is present, oxygen radical is generated, and a molded body consisting of a hydrophobic polymer is thereby decomposed. Accordingly, the oxygen concentration around the molded body is desirably 10 vol % or less during irradiation.

Since the separation membrane according to the eighth embodiment has a high adhesion-suppressing property, it can be preferably used as a separation membrane for water treatment or a separation membrane for biological components. Moreover, the modification method according to the present embodiment can be applied, not only to membranes, but also to various types of molded bodies, and it can easily carry out surface modification at a high efficiency. This surface modification method is particularly suitable for a blood purification module. The blood purification module herein means a module having a function to circulate blood to the outside of the body and to remove waste products or harmful substances from the blood. Examples of such a module include an artificial kidney and an exotoxin adsorption column.

Hereinafter, the present invention will be described more specifically in the following examples.

### Example 1

### [Comparison of migration factors effective for migration of dental pulp stem cells by TaxiScan]

For real-time horizontal chemotaxis analysis, the 4th-generation dog dental pulp stem cells CD31⁻SP were used. Using TAXIScan-FL (Effector Cell Institute, Tokyo), a channel optimized to the size of cells (8 µm) was formed between silicone having pores with a pore size of 6 µm and a glass plate, and 1 µl of cells (10⁵ cells/ml) was then poured in one side of the channel. Various types of migration factors (10 ng/µl) were each poured into the opposite side thereof, so as to form a certain concentration gradient. Based on video images of migration, the number of migrating cells was counted every 30 minutes until 4 hours after initiation of the operation. Fig. 6(a) shows a difference in migration ability depending on the types of the migration factors over time. In the case of BDNF, the dental pulp stem cells migrated very promptly, and 2 hours later, the migration level reached plateau. In the case of SDF-1 and bFGF as well, migration progressed relatively promptly. In the case of GDNF, VEGF, MMP3, and G-CSF, the number of the migrating cells was gradually increased, and 4 hours later, the number of migrating cells became almost the same, except for PDGF and GM-CSF.

### [Concentration of migration factor effective for migration of dental pulp stem cells, analyzed by TaxiScan]

1 µl of the dog dental pulp stem cells, CD31⁻ SP (10⁵ cells/ml) at the 4th passage of culture was poured in a TAXIScan-FL microchannel, and 1 µl each of G-CSF was then poured in the opposite side in a concentration of 0, 0.1, 1, 5, 10, 20, 40, or 100 ng/µl. Based on video images of migration, the number of migrating cells was counted every 30 minutes until 1.5 hours after initiation of the operation. Fig. 6(b) shows a difference in migration ability depending on the concentration of G-CSF over time. In the case of 10 ng/µl G-CSF, the number of migrating cells is largest, and then, in the order of the concentration of 5, 40, 20, 100, 1, and 0.1 ng/µl, the number of migrating cells was decreased.

### [Concentration of serum effective for migration of dental pulp stem cells, analyzed by TaxiScan]

1 µl of human dental pulp stem cells (10⁵ cells/ml) was poured in a TAXIScan-FL microchannel, and human serum was then poured in the opposite side in a concentration of 0%, 5%, 10%, 15%, or 20%. Based on video images of migration, the number of migrating cells was counted every 3 hours until 24 hours after initiation of the operation. Thereafter, a comparison was made on migration ability, in the case of using 10% and 20% human serums and 100 ng/ml G-CSF and SDF-1. Fig. 7(a) shows a difference in migration ability depending on the concentration of serum over time. The number of migrating cells was largest in the case of 20% human serum, and then, in the order of the concentration of 15%, 10%, and 5%, the number of migrating cells was decreased. Fig. 7(b) shows migration ability in the case of G-CSF or SDF-1 over time. The number of migrating cells in the case of G-CSF or SDF-1 was greater than the number of migrating cells in the case of 20% human serum.

### [Separation of dental pulp stem cells]

There was assembled a membrane separation culture device composed of: a PET-made upper structure having a bottom surface with a diameter of 6.4 mm, an opening portion with a diameter of 11.0 mm, and a height of 17.5 mm, wherein a non-cell-adhesively coated PET membrane (2 × 10⁵ pores/cm², pore size: 3 µm) of Cell culture Insert was equipped into the bottom surface thereof; and a polystyrene-made lower structure having a bottom surface with a diameter of 15.0 mm, an opening portion with a diameter of 15.0 mm, and a height of 22.0 mm. In order to impart a cell adhesion-suppressing property to the surface of the PET-membrane, the PET membrane had previously been immersed in an aqueous solution prepared by adding 0.1% ethanol to a 1,000 ppm aqueous solution of a polyvinyl pyrrolidone-polyvinyl acetate copolymer (vinyl pyrrolidone/vinyl acetate (6/4) copolymer ("Kollidon VA64," manufactured by BASF)), so as to seal it. Thereafter, the membrane was modified by irradiation with a γ-ray (25 kGy), thereby preparing a separation membrane. On this membrane of the upper structure, fresh dog primary dental pulp cells were dispersed at a cell density of 1 × 10⁵ cells/250 µl. On the other hand, G-CSF or SDF-1 was added into Dulbecco's modified Eagle's medium (DMEM) containing 10% dog serum in the lower structure, resulting in a final concentration of 100 ng/ml. Six hours later, the medium was replaced with fresh medium, and the G-CSF was then removed. The resultant was further cultured in DMEM containing 10% dog serum. Fig.s 8(a), (b) and (c) each show a phase contrast microscopic image of dental pulp stem cells, which have migrated, adhered, and further proliferated. It became clear that, in all of the migration factors, star-like cells having projections adhered and proliferated, as in the case of separating CD31⁻ SP cells, CD105⁺ cells or CXCR4⁺ cells by flow cytometry.

### [Characterization of separated dental pulp stem cells]

The above described dental pulp stem cells, which had been membrane-separated using G-CSF and SDF-1 in the membrane separation culture device, were subcultured for three generations. Thereafter, the cells were dispersed in DMEM containing 2% serum at a cell density of 1 × 10⁶ cells/ml, and were then labeled with various types of stem cell surface antigen marker antibodies (CD29, CD31, CD34, CD44, CD73, CD90, CD105, CD146, CD150, and CXCR4) at 4°C for 30 minutes. Thereafter, flow cytometry was carried out. That is to say, the cells were labeled at 4°C for 90 minutes using mouse IgG1 negative control (AbD Serotec Ltd.), mouse IgG1 negative control (fluorescein isothiocyanate, FITC) (MCA928F) (AbD Serotec), mouse IgG1 negative control (Phycoerythrin-Cy5, PE-Cy5) (MCA928C) (AbD Serotec), mouse IgG1 negative control (Alexa 647) (MRC OX-34) (AbD Serotec), antibodies to the following: CD29 (PE-Cy5) (MEM-101A) (eBioscience), CD31 (FITC) (Qbend10) (Dako), CD34 (Allophycocyanin,APC) (1H6) (R&D Systems,Inc.), CD44 (Phycoerythrin-Cy7,PE-Cy7) (IM7) (eBioscience),CD73 (APC) (AD2) (BioLegend),CD90 (FITC) (YKIX337.217) (AbD Serotec), anti-human CD105 (PE) (43A3) (BioLegend)CD146 (FITC) (sc-18837) (Santa Cruz,Biotech,Santa Cruz,CA,USA),CD150 (FITC) (A12) (AbD Serotec), CXCR4 (FITC) (12G5) (R&D). As a control, dog dental pulp CD105⁺ cells, which had been separated by flow cytometry, were used.

Table 1 shows the expression of surface antigens on the 3rd-generation dental pulp stem cells, which had been separated and cultured in the above-described membrane separation culture device, analyzed by flow cytometry. As with dog dental pulp CD105⁺ cells, the CD105-positive expression rate of the cells separated in the membrane separation culture device was 95.1% in the case of using G-CSF, and it was 89.5% in the case of using SDF-1. In addition, the CD29-, CD44-, CD73-, and CD90-positive expression rates of the cells were 95% or more in both fractions, and thus, it was considered that large quantities of stem cells and/or precursor cells were contained therein. Moreover, the CXCR4-positive expression rate of the cells was a half of that in the case of the dog dental pulp CD105⁺ cells separated by flow cytometry, and further, the cells were almost negative to CD31 and CD146.

**[Table 1]**

| | membrane-separated dental pulp stem cells | dental pulp CD105⁺ cells |
|---|---|---|
| **CD24** | **1.5%** | **1.8%** |
| **CD29** | **99.6%** | **95.9%** |
| **CD31** | **0.2%** | **0.0%** |
| **CD33** | **6.8%** | **3.7%** |
| **CD34** | **48.6%** | **45.5%** |
| **CD44** | **100%** | **96.2%** |
| **CD73** | **93.3%** | **97.2%** |
| **CD90** | **92.0%** | **98.1%** |
| **CD105** | **95.1%** | **98.5%** |
| **CD146** | **0%** | **0.8%** |
| **CD150** | **0.6%** | **2.3%** |
| **MHC class I** | **70.9%** | **36.0%** |
| **MHC class II** | **3.4%** | **0.4%** |
| **CXCR4** | **5.3%** | **12.2%** |

Subsequently, using Trizol (Invitrogen), total RNA was separated from the 3rd-generation dental pulp stem cells that had been membrane-separated using G-CSF. Thereafter, first-strand cDNA was synthesized using ReverTra Ace-α (Toyobo), and it was then labeled with Light Cycler-Fast Start DNA master SYBR Green I (Roche Diagnostics). Thereafter, real-time RT- PCR was performed for stem cell markers (CXCR4, Sox2, Stat3, and Bmi1) employing Light Cycler (Roche Diagnostics) in accordance with a program of 95°C-10 seconds, 62°C-15 seconds, and 72°C-8 seconds. Further, as angiogenesis-inducing factors and neurotrophic factors, matrix metalloproteinase (MMP)-3, VEGF-A, granulocyte-monocyte colony-stimulating factor (GM-CSF), NGF, and BDNF were used. As controls, dental pulp CD105⁺ cells and unseparated dental pulp test cells were used, and primers used as they were standardized with β-actin.

Table 2 shows primers used in the real-time RT-PCR analysis of stem cell markers, angiogenesis-inducing factors, and neurotrophic factors.

**[Table 2]**

| Canine primers for real-time reverse transcription-polymerase chain reaction | | | | |
|---|---|---|---|---|
| Gene | | 5'←DNA Sequence→3' | product size | Accession number |
| *Sox2* | Forward | AGCTAGTCTCCAAGCGACGA(SEQ ID NO. 1) | 193bp | XM_545216 |
| | Reverse | CCACGTTTGCAACTGTCCTA(SEQ ID NO. 2) | | |
| *Bmi1* | Forward | CACTCCCGTTCAGTCTCCTC(SEQ ID NO. 3) | 150np | XM_544225 |
| | Reverse | CCAGATGAAGTTGCTGACGA(SEQ ID NO. 4) | | |
| *CXCR4* | Forward | CTGTGGCAAACTGGTACTTC(SEQ ID NO. 5) | 210bp | NM_001048026 |
| | Reverse | TCAACAGGAGGGCAGGTATC(SEQ ID NO. 6) | | |
| *Stat3* | Forward | GTGGTGACGGAGAAGCAACA(SEQ ID NO. 7) | 191bp | XM_844672 |
| | Reverse | TTCTGTCTGGTCACCGACTG(SEQ ID NO. 8) | | |
| *GM-CSF* | Forward | GCAGAACCTGCTTTTCTTGG(SEQ ID NO. 9) | 195bp | S49738 |
| | Reverse | CCCTCAGGGTCAAACACTTC(SEQ ID NO. 10) | | |
| *MMP3* | Forward | CCCTCTGATTCCTCCAATGA(SEQ ID NO. 11) | 210bp | AY183143 |
| | Reverse | GGATGGCCAAAATGAAGAGA(SEQ ID NO. 12) | | |
| *VEGFA* | Forward | CTACCTCCACCATGCCAAGT(SEQ ID NO. 13) | 183bp | NM_001003175 |
| | Reverse | ACGCAGGATGGCTTGAAGAT(SEQ ID NO. 14) | | |
| *BDNF* | Forward | GTTGGCCGACACTTTTGAAC(SEQ ID NO. 15) | 202bp | NM_001002975 |
| | Reverse | CCTCATCGACATGTTTGCAG(SEQ ID NO. 16) | | |
| *GDNF* | Forward | GCCGAGCAGTGACTCAAAC(SEQ ID NO. 17) | 104bp | XM_546342 |
| | Reverse | TCTCGGGTGACCTTTTCAG(SEQ ID NO. 18) | | |
| *NGF* | Forward | CAACAGGACTCACAGGAGCA(SEQ ID NO. 19) | 156bp | XM_540250 |
| | Reverse | ATGTTCACCTCTCCCAGCAC(SEQ ID NO. 20) | | |
| *β -actin* | Forward | AAGTACCCCATTGAGCACGG(SEQ ID NO. 21) | 257bp | Z70044 |
| | Reverse | ATCACGATGCCAGTGGTGCG(SEQ ID NO. 22) | | |

Table 3 shows the expression levels of the mRNAs of stem cell markers, angiogenesis-inducing factors, and neurotrophic factors in the 3rd-generation membrane-separated dog dental pulp stem cells separated using G-CSF and the dental pulp CD105⁺ cells, analyzed by real time RT-PCR, which were compared with dental pulp test cells. The angiogenesis-inducing factor VEGF and the neurotrophic factor GDNF exhibited almost the same expression levels in the two types of cells. The expression levels of GM-CSF and MMP3 were higher in the membrane-separated cells than in the CD105⁺ cells by 10 times or more. On the other hand, the expression levels of BDNF and NGF were higher in the CD105-positive cells than in the membrane-separated cells. The expression levels of the stem cell markers CXCR4 and Bmi1 were much higher in the membrane-separated cells than in the CD105⁺ cells. The expression level of Stat3 was almost the same in the two types of cells, and the expression level of Sox2 was slightly lower than in the membrane-separated cells than in the CD105⁺ cells.

**[Table 3]**

| | membrane-separated dental pulp stem cells/unseparated dental pulp test cells | dental pulp CD105⁺ cells/unseparated dental pulp test cells |
|---|---|---|
| Sox2 | 20.7 | 64.0 |
| Bmi1 | 29.9 | 3.5 |
| CXCR4 | 8 | 16.8 |
| Stat3 | 1.2 | 0.8 |
| GM-CSF | 53.2 | 5.8 |
| MMP3 | 313.4 | 26.1 |
| VEGF | 3.8 | 3.6 |
| BDNF | 1.3 | 16.0 |
| GDNF | 4.1 | 4.2 |
| NGF | 1.8 | 4.1 |

### [Pluripotency in vitro]

Membrane-separated dental pulp cells were induced to differentiate into blood and nerve for a period from the 3rd generation to the 5th generation. The results are shown in Fig. 9. As shown in Fig. 9(a), the membrane-separated dental pulp stem cells exhibited ability to differentiate into vascular endothelial cells. In addition, as shown in Fig. 9(b), the membrane-separated dental pulp stem cells exhibited neurosphere formation.

### Example 2

### [Regeneration of dental pulp by autologous transplantation of membrane-separated dental pulp stem cells into root canal after extirpation of dental pulp]

There was established an experimental model, in which the dental pulp was completely removed from the root apex-completely-formed permanent teeth of a dog (Narc, Chiba, Japan), and a cellular fraction was transplanted therein to regenerate dental pulp. The dog was undergone general anesthesia with sodium pentobarbital (Schering-Plough, Germany), and the dental pulp was completely removed from the maxillary second incisor tooth and mandibular third incisor tooth of the dog, and the root apex portion was enlarged to a size of 0.7 mm using #70K-file (MANI, INC., Tochigi, Japan). The membrane-separated dental pulp stem cells of Example 2 were transplanted into the root apex side, and G-CSF was transplanted into the dental crown side. Specifically, the 4th-generation membrane-separated dental pulp stem cells (5 × 10⁵ cells), together with collagen TE (Nitta Gelatin, Osaka, Japan), were labeled with DiI, and they were then autologously transplanted into the lower portion in the root canal. Further, into the upper portion of the root canal, G-CSF (final concentration: 15 ng/µl) together with collagen TE was transplanted. The cavity was treated with zinc phosphate cement (Elite Cement, GC, Tokyo, Japan) and a bonding material (Clearfil Mega Bond, Kuraray), and was then repaired with a composite resin (Clearfil FII, Kuraray, Kurashiki, Japan). As a control, dental pulp CD105-positive cells were used. Fourteen days later, a specimen was prepared. For morphology analysis, the specimen was immobilized with 4% paraformaldehyde (PFA) (Nakarai Tesque, Kyoto, Japan) at 4°C overnight. Thereafter, the specimen was decalcified with 10% formic acid and was then embedded in paraffin wax (Sigma). A paraffin section (thickness: 5 µm) was stained with hematoxylin-eosin (HE), and was then morphologically observed.

Fig. 10(a) is a view showing regeneration of the dental pulp by autologous transplantation of G-CSF and membrane-separated dental pulp stem cells. Fig. 10(b) is an enlarged view of an area (b) enclosed with a square in Fig. 10(a). Fig. 10(c) is an enlarged view of an area C enclosed with a square in Fig. 10(a). As shown in Fig. 10(a), Fig. 10(b) and Fig. 10(c), when the membrane-separated dental pulp stem cells are transplanted together with G-CSF, dental pulp-like tissues were formed until the 14th day after completion of the transplantation. As shown in Fig. 10(b), cells in the regenerated tissues have a fusiform or star-like shape, and they were similar to cells in normal dental pulp tissues (Fig. 10(d)). As shown in Fig. 10(c), odontoblast-like cells adhered to the dentin wall of the root canal and extended their projections into canaliculi.

### Example 3

### [Membrane-separated human dental pulp stem cells]

Using a membrane separation culture device (1 × 10⁵ pores/cm², pore size: 8 µm), fresh human primary dental pulp cells (1 × 10⁵ cells/100 µl) were dispersed on the upper portion of the membrane. 10 ng/ml or 100 ng/ml G-CSF was added into 10% human serum alone or in DMEM containing 10% human serum in the lower structure of the membrane. Twenty-two hours later, the medium was replaced with fresh medium, the G-CSF was then removed, and the resultant was further cultured in DMEM containing 10% human serum. Fig. 11 shows a phase contrast microscopic image of dental pulp stem cells that have migrated, adhered, and further proliferated. As shown in Fig.s 12(a), (b), and (c), it became clear that, in all of 10% human serum, 10 ng/ml G-CSF, and 100 ng/ml G-CSF, star-like cells having projections adhered, and that these cells proliferated, as in the case of separating CD31⁻SP cells, CD105⁺ cells or CXCR4⁺ cells by flow cytometry (Fig.s 12 (d) and (e)).

### Example 4

### [Membrane-separated tissue stem cells]

Using the membrane separation culture device according to the present invention (1 × 10⁵ pores/cm², pore size: 8 µm), pig dental pulp cells, bone marrow cells, and adipose cells (1 × 10⁵ cells/100pul) were dispersed on the upper portion of the membrane. Meanwhile, 100 ng/ml G-CSF was added into DMEM containing 10% fetal bovine serum in the lower structure of the membrane. Twenty-two hours later, the medium was replaced with fresh medium, the G-CSF was then removed, and the resultant was further cultured in DMEM containing 10% fetal bovine serum. As shown in Fig.s 13(a), (b), and (c), it became clear that, in all of the dental pulp, bone marrow, and adipose cells, star-like cells having projections adhered, and that as shown in Fig. 13(d), these cells proliferated, as in the case of separating CD31⁻SP cells or CD105⁺ cells by flow cytometry.

### Example 5

### [1. Comparison of migration factors effective for migration of dental pulp stem cells, analyzed by TAXIScan]

Using TAXIScan-FL (Effector Cell Institute, Tokyo), real-time horizontal chemotaxis analysis was carried out on unseparated human dental pulp cells. A channel optimized to the size of cells (8 µm) was formed between silicone having pores with a pore size of 6 µm and a glass plate, and 1 µl of cells (10⁵ cells/ml) was then poured in one side of the channel. Various types of migration factors (10 ng/µl; BDNF, GDNF, NGF, PDGF, G-CSF, SDF-1, bFGF, VEGF, LIF and GM-CSF) were each poured in an amount of 1µl into the opposite side thereof, so as to form a certain concentration gradient. Based on video images of migration, the number of migrating cells was counted every 3 hours until 15 hours after initiation of the operation. Moreover, using G-CSF and bFGF as migration factors, a change in migration ability in the case of adding 10% fetal bovine serum was examined.

The measurement results of migration ability in the case of using various types of migration factors are shown in Fig. 14. Seeing a difference, over time, in migration ability depending on various migration factors, large quantities of migrating cells were observed when BDNF, GDNF, NGF, PDGF and G-CSF were used. In the case of using SDF-1, bFGF and LIF as well, relatively large quantities of migrating cells were observed. In the case of using GM-CSF, however, migration of cells was hardly observed. As such, a comparative review was made between the migration factors G-CSF and bFGF, whose agents satisfying clinically used standards (Good Manufacturing Practice (GMP)) can be easily obtained and which had already received pharmaceutical approval in Japan.

The measurement results of migration ability obtained using G-CSF and bFGF are shown in Fig. 15. When G-CSF and bFGF were added, the migration ability of cells became higher than that in the case of adding only 10% serum. When 10% serum was added to each of G-CSF and bFGF, the migration of the cells was further promoted. In particular, it became clear that G-CSF promotes the migration of cells more strongly than bFGF does. From these results, it was found that it is effective for carrying out membrane separation of migrating cells by adding 10% serum to G-CSF.

As described above, with regard to the expression of a stem cell marker, the membrane-separated cells that had been separated using 10 ng/ml G-CSF had a stem cell marker expression rate similar to that of the CD105⁺ cells separated by flow cytometry. The membrane-separated cells that had been separated using 100 ng/ml G-CSF had the expression rates of CXCR4 and G-CSFR that were higher than those of the CD105⁺ cells, and thus, it has been suggested that the membrane-separated cells might contain larger quantities of stem cells. Moreover, the membrane-separated cells that had been separated using 100 ng/ml G-CSF had cell proliferative ability and migration ability that were higher than those of the CD105⁺ cells. Furthermore, with regard to the expression of mRNAs of angiogenic factors and neurotrophic factors as well, the membrane-separated cells separated using 100 ng/ml G-CSF exhibited the highest expression levels. The present inventors had already reported that dental pulp CD105⁺ cells have high angiogenesis-inducing ability, high nerve-inducing ability, and high ability to regenerate the dental pulp. However, it has been suggested from the results of the present experiment that the membrane-separated cells separated using 100 ng/ml G-CSF are also effective for regeneration of the dental pulp/dentin, as in the case of the CD105⁺ cells.

### [2. Number of cells dispersed for separation of dental pulp stem cells using membrane separation device]

Cellculture Insert (polycarbonate base material membrane (Polycarbonate Membrane) Transwell (registered trademark) Inserts; 2 × 10⁵ pores/cm², pore size: 8 µm, diameter of bottom surface: 6.4 mm, diameter of opening portion: 11.0 mm, height: 17.5 mm) (Corning), used an upper structure, was inserted into a 24-well plate (diameter: 15.0 mm, diameter of opening portion: 15.0 mm, height: 22.0 mm) (Falcon), used as a lower structure, and the thus prepared device was used as a membrane separation device. In order to impart a non-cell-adhesive property to the polycarbonate base material membrane, the membrane had previously been immersed in an aqueous solution prepared by adding 0.1% ethanol to a 1000 ppm aqueous solution of a polyvinyl pyrrolidone-polyvinyl acetate copolymer (vinyl pyrrolidone/vinyl acetate (6/4) copolymer ("Kollidon VA64," manufactured by BASF)), so as to seal it. Thereafter, the resulting membrane was modified by irradiation with a γ-ray (25 kGy), thereby preparing a separation membrane. As a result of the aforementioned operation, the polyvinyl pyrrolidone-polyvinyl acetate copolymer was bound to the surface of the base material membrane via a covalent bond. However, the polymer had high safety, and also, the ethanol used simultaneously with the copolymer was decomposed by irradiation with the γ-ray and the concentration thereof was reduced. Accordingly, this surface modification was highly safe. The 2nd-generation human dental pulp cells were dispersed on the upper portion of this separation membrane at a cell density of 2 × 10⁴ cells/100 µl, 1 × 10⁵ cells/100 µl. Meanwhile, G-CSF (final concentration: 100 ng/ml) was added into Dulbecco's modified Eagle's medium (DMEM) containing 10% human serum in 24 wells of the lower structure of the membrane. Forty-eight hours later, the G-CSF was removed, and the medium was replaced with another DMEM containing 10% human serum. Thereafter, the number of cells adhered to the lower portions of 24 wells was measured under phase contrast microscope.

As a result, the separated cell percentage was 5% at a cell density of 2 × 10⁴ cells/100 µl, and was 1% at a cell density of 1 × 10⁵ cells/100 µl. These results suggested that cell separation efficiency be different depending on the number of cells.

### [3. Action time of migration factors for separation of dental pulp stem cells using membrane separation device]

The 2nd-generation human dental pulp cells were dispersed on the upper portion of the separation membrane of a membrane separation device at a cell density of 2 × 10⁴ cells/100 µl. Meanwhile, G-CSF (final concentration: 100 ng/ml) was added into DMEM containing 10% human serum in 24 wells of the lower structure of the membrane. Thereafter, 12, 24, 48, and 72 hours later, the number of cells adhered to the lower portions of the 24 wells was counted under a phase contrast microscope.

The action times of G-CSF were set at 12, 24, 48, and 72 hours, and the number of adhering cells was counted in each time point. As a result, 12 hours later, the separated cell percentage was 0.3%, 24 hours later, it was 1.7%, 48 hours later, it was 5%, and 72 hours later, it was also 5%.

### [4. Concentration of G-CSF for separation of dental pulp stem cells using membrane separation device]

The 2nd-generation human dental pulp cells were dispersed on the upper portion of the separation membrane of a membrane separation device at a cell density of 2 × 10⁴ cells/100 µl. Meanwhile, G-CSF was added into Dulbecco's modified Eagle's medium (DMEM) containing 10% human serum in 24 wells of the lower structure of the membrane to final concentrations of 0, 10, 100, and 500 ng/ml. Forty-eight hours later, the G-CSF was removed, and the medium was then replaced with another DMEM containing 10% human serum. Then, the number of cells adhered to the lower portions of the 24 wells was counted under phase contrast microscope. Thereafter, the cells were further cultured, and after they had become 70% confluent, they were subcultured.

The above described dental pulp stem cells, which had been membrane-separated using various concentrations of G-CSF, were subcultured to the 6th generation. Thereafter, the expression rates of the stem cell surface antigen markers were measured by flow cytometry. Specifically, the cells were dispersed in DMEM containing 2% serum at a cell density of 1 × 10⁶ cells/ml, and were then labeled with stem cell marker antibodies at 4°C for 30 minutes. Specifically, the cells were labeled at 4°C for 90 minutes using mouse IgG1 negative control (AbD Serotec Ltd.), hamster IgG (PE-cy7) (eBio299Arm) (eBioscience), rat IgG2b (PE-cy7) (RTK4530) (Biolegend), mouse IgG1 (APC) (NOPC-21) (Biolegend), mouse IgG1 (RPE) (SFL928PE) (AbD Serotec), mouse IgG1 (Alexa647) (F8-11-13) (AbD Serotec), mouse IgG2a (FITC) (S43.10) (MACS), mouse IgG1 (FITC) (MOPC-21) (Biolegend), antibodies to the following: CD29 (Phycoerythrin, PE-cy7) (eBio299Arm) (eBioscience), CD31 (PE) (WM59) (BD Pharmingen), CD44 (PE-cy7) (IM7) (eBioscience), CD73 (APC) (AD2) (Biolegend), CD90 (Alexa647) (F15-42-1) (AbD Serotec), CD105 (PE) (43A3) (Biolegend), CD146 (Alexa647) (OJ79c) (AbD Serotec), CXCR4 (FITC) (12G5) (R&D Systems), G-CSFR (CD114) (FITC) (38660). As controls, human dental pulp CD105⁺ cells separated by flow cytometry and unseparated human dental pulp test cells were used.

Subsequently, using Trizol (Invitrogen), total RNA was separated from the 6th-generation membrane-separated cells separated using various concentrations of G-CSF. Thereafter, first-strand cDNA was synthesized using ReverTra Ace-α (Toyobo), and it was then labeled with Light Cycler-Fast Start DNA master SYBR Green I (Roche Diagnostics). Thereafter, real-time RT- PCR was performed for angiogenesis-inducing factors and neurotrophic factors, employing Light Cycler (Roche Diagnostics), in accordance with a program of 95°C-10 seconds, 65°C-15 seconds, and 72°C-8 seconds. As angiogenesis-inducing factors and neurotrophic factors, the primers of granulocyte-monocyte colony-stimulating factor (GM-CSF), matrix metalloproteinase (MMP)-3, VEGF-A, brain-derived neurotrophic factor (BDNF), nerve growth factor (NGF), and neurotrophin-3 (NT-3) were used (Table 4). As controls, dental pulp CD105⁺ cells and unseparated human dental pulp test cells were used, and they were standardized with β-actin.

**[Table 4]**

| Gene | | 5'←DNA Sequence→3' | product size | Accession number |
|---|---|---|---|---|
| Oct4 | Forward | CAGTGCCCGAAACCCACAC(SEQ ID NO. 23) | 161 | NM_002701 |
| | Reverse | GGAGACCCAGCAGCCTCAAA(SEQ ID NO. 24) | | |
| Nanog | Forward | CAGAAGGCCTCAGCACCTAC(SEQ ID NO. 25) | 111 | NM_024865 |
| | Reverse | ATTGTTCCAGGTCTGGTTGC(SEQ ID NO. 26) | | |
| Sox2 | Forward | AATGCCTTCATGGTGTGGTC(SEQ ID NO. 27) | 203 | NM_003106 |
| | Reverse | CGGGGCCGGTATTTATAATC(SEQ ID NO. 28) | | |
| Rex1 | Forward | TGGACACGTCTGTGCTCTTC(SEQ ID NO. 29) | 168 | BC032244 |
| | Reverse | CTCGAACCTTCCAGATCACC(SEQ ID NO. 30) | | |
| Stat3 | Forward | GTGGTGACGGAGAAGCAGCA(SEQ ID NO. 31) | 191 | NM_139276 |
| | Reverse | TTCTGCCTGGTCACTGACTG(SEQ ID NO. 32) | | |
| CXCR4 | Forward | CCGTGGCAAACTGGTACTTT(SEQ ID NO. 33) | 210 | NM_001008540 |
| | Reverse | TCAGCAGGAGGGCAGGGATC(SEQ ID NO. 34) | | |
| GM-CSF | Forward | GCCTGGAGCTGTACAAGCAG(SEQ ID NO. 35) | 193 bp | NM_000758 |
| | Reverse | CAGCAGTCAAAGGGGATGAC(SEQ ID NO. 36) | | |
| MMP3 | Forward | CCTCAGGAAGCTTGAACCTG(SEQ ID NO. 37) | 192 bp | NM_002422 |
| | Reverse | GGGAAACCTAGGGTGTGGAT(SEQ ID NO. 38) | | |
| VEGFA | Forward | ATGGCAGAAGGAGACCAGAA(SEQ ID NO. 39) | 224 bp | NM_001033756 |
| | Reverse | ATGGCGATGTTGAACTCCTC(SEQ ID NO. 40) | | |
| BDNF | Forward | AAACATCCGAGGACAAGGTG(SEQ ID NO. 41) | 202 bp | NM_170735 |
| | Reverse | CGTGTACAAGTCTGCGTCCT(SEQ ID NO. 42) | | |
| GDNF | Forward | CCAACCCAGAGAATTCCAGA(SEQ ID NO. 43) | 150 bp | NM_000514 |
| | Reverse | AGCCGCTGCAGTACCTAAAA(SEQ ID NO. 44) | | |
| NGF | Forward | ATACAGGCGGAACCACACTC(SEQ ID NO. 45) | **181** bp | NM_002506 |
| | Reverse | GCCTGGGGTCCACAGTAAT(SEQ ID NO. 46) | | |
| NT-3 | Forward | AGACTCGCTCAATTCCCTCA(SEQ ID NO. 47) | 187 bp | BC107075 |
| | Reverse | GGTGTCCATTGCAATCACTG(SEQ ID NO. 48) | | |
| *β* -actin | Forward | GGACTTCGAGCAAGAGATGG(SEQ ID NO. 49) | 234 bp | NM_001101 |
| | Reverse | AGCACTGTGTTGGCGTACAG(SEQ ID NO. 50) | | |

Furthermore, the 7th-generation membrane-separated dental pulp cells were induced to differentiate into blood, nerve, adipose, dentin, and bone *in vitro* according to an ordinary method. Further, membrane-separated human cells that had been separated with various concentrations of G-CSF were compared with one another, in terms of cell proliferative ability by stimulation with 10% human serum or 100 ng/ml G-CSF, and cell migration ability with 10% human serum or 100 ng/ml G-CSF.

Dental pulp stem cells, which had adhered to the plate and had further proliferated, were observed from phase contrast microscopic images. With regard to unseparated 6^{th}-generation human dental pulp test cells, 6th-generation human dental pulp CD105⁺ cells, membrane-separated cells that had been membrane-separated only with 10% serum and had been then cultured for 7 days, membrane-separated cells that had been membrane-separated with 10 ng/ml G-CSF + 10% serum and had been then cultured for 7 days, membrane-separated cells that had been membrane-separated with 100 ng/ml G-CSF + 10% serum and had been then cultured for 7 days, and membrane-separated cells that had been membrane-separated with 500 ng/ml G-CSF + 10% serum and had been then cultured for 7 days, phase contrast microscopic images were obtained. Even in a case in which any of the concentrations, such as 10 % human serum, or 0 ng/ml, 10 ng/ml, 100 ng/ml or 500 ng/ml G-CSF, is used, adhesion and proliferation of star-like cells having projections were observed. The number of adhering cells was counted. As a result, in the case of 100 ng/ml G-CSF, the separated cell percentage was 5%, and then, in the case of 500 ng/ml G-CSF, it was 4%, in the case of 10 ng/ml G-CSF, it was 3%, and in the case of 0 ng/ml G-CSF, it was 2%.

Subsequently, the analysis of stem cell surface markers by flow cytometry is shown in Table 5. The stem cell marker expression rates were compared by flow cytometry. As a result, CD29, CD44, CD73, and CD90 were all positive, and no difference was found. The expression rate of CD105 was 19% in unseparated human dental pulp test cells, but it was 90% or more in the membrane-separated cells separated with 10 ng/ml and 100 ng/ml G-CSF, as in the case of the control CD105⁺ cells. Moreover, in the membrane-separated cells separated with 500 ng/ml and 0 ng/ml G-CSF, the expression rates of CD105 were low levels, which were 67% and 58%, respectively. The expression rate of CXCR4 was 4.5% in unseparated human dental pulp test cells, was 8% in the CD105⁺ cells, and was 5% in the membrane-separated cells separated with 0 ng/ml G-CSF, which were all low levels. In contrast, the expression rate of CXCR4 was highest (15%) in the membrane-separated cells separated with 100 ng/ml G-CSF, and was 10% or more in the membrane-separated cells separated with 10 ng/ml and 500 ng/ml G-CSF. Furthermore, the expression rate of G-CSFR as a G-CSF receptor was 18% in CD105⁺ cells, and was the highest (76%) in the membrane-separated cells separated with 100 ng/ml G-CSF. Thus, a reduction in the expression rate was found in the order of 500 ng/ml G-CSF and 10 ng/ml G-CSF. From these results, it has been suggested that membrane-separated cells separated with 100 ng/ml G-CSF, in which the positive expression rates of CD105, CXCR4, and G-CSFR are the highest, might contain the greatest quantities of stem cells and/or precursor cells.

**[Table 5]**

| | unseparated dental pulp test cells | membrane-separated cells | | | | dental pulp CD105⁺ cells |
|---|---|---|---|---|---|---|
| | | G-CSF 0 ng/ml | G-CSF 10 ng/ml | G-CSF 100 ng/ml | G-CSF 500 ng/ml | |
| CD29 | 94.9 % | 97.8% | 94.9 % | 96.9 % | 97.4 % | 95.6 % |
| CD31 | 0.0 % | 0.0% | 0.2 % | 0.0 % | 0.3 % | 0.4 % |
| CD44 | 97.2 % | 98.3% | 98.0 % | 94.8 % | 94.8 % | 94.1 % |
| CD73 | 99.0 % | 90.6% | 99.5 % | 99.2 % | 99.3 % | 97.1 % |
| CD90 | 99.4 % | 97.6% | 99.5 % | 99.4 % | 99.0 % | 99.6 % |
| CD105 | 18.9 % | 58.3% | 94.0 % | 98.1 % | 66.9 % | 96.8 % |
| CD146 | 14.6 % | 13.3 % | 16.2 % | 9.2 % | 16.7 % | 13.0 % |
| CXCR4 | 4.5 % | 5.1% | 12.1 % | 15.3 % | 10.2 % | 7.8 % |
| G-CSFR | 9.3 % | 14.5% | 28.6 % | 75.9 % | 32.5 % | 18.0 % |

The pluripotency of membrane-separated cells that had been separated using 100 ng/ml G-CSF was studied. Specifically, the angiogenesis-inducing ability and nerve-inducing ability of the membrane-separated cells that had been separated using G-CSF (concentration: 100 ng/ml) were examined based on a phase contrast microscopic image. As in the case of CD105⁺ cells, 6 hours later, the membrane-separated cells formed a cord-like structure on matrigel, and thus, the cells showed ability to differentiate into vascular endothelial cells. In the case of unseparated human dental pulp test cells, such formation of a cord-like structure was not seen even if it was observed for a long period of time. Moreover, the membrane-separated cells separated with 100 ng/ml G-CSF were found to form neurospheres on the 14th day of induction, as in the case of CD105⁺ cells, although such formation of neurospheres was hardly seen in unseparated human dental pulp test cells. The adipose-inducing ability of the membrane-separated cells separated with G-CSF (concentration: 100 ng/ml) was examined based on an optical microscopic image. Adipose induction was observed in all of cellular fractions. The expression level of adipose marker mRNA was higher in these membrane-separated cells than in unseparated human dental pulp test cells. The bone/dentin-inducing ability of the membrane-separated cells separated with G-CSF having an extremely preferred concentration (100 ng/ml) was examined based on an optical microscopic image. Such bone/dentin induction was also observed in all of cellular fractions. The expression level of bone/dentin marker mRNA was lower in the membrane-separated cells than in unseparated human dental pulp test cells.

The results obtained by analyzing the expression of mRNAs of angiogenesis-inducing factors and neurotrophic factors by real-time RT-PCR are shown in Table 6.

**[Table 6]**

| | unseparated dental pulp test cells | membrane-separated cells | | | | dental pulp CD105⁺ cells |
|---|---|---|---|---|---|---|
| | | G-CSF 0 ng/ml | G-CSF 10 ng/ml | G-CSF 100 ng/ml | G-CSF 500 ng/ml | |
| Oct4 | 1.0 | 1.1 | 1.3 | 2.0 | 1.3 | 1.6 |
| Nanog | 1.0 | 1.1 | 1.3 | 1.9 | 1.3 | 2.1 |
| Sox2 | 1.0 | 0.7 | 26.0 | 27.7 | 19.6 | 40.8 |
| Rex1 | 1.0 | 1.7 | 2.8 | 3.0 | 1.5 | 1.6 |
| Stat3 | 1.0 | 0.7 | 1.3 | 1.9 | 1.2 | 1.0 |
| CXCR4 | 1.0 | 1.5 | 21.6 | 35.5 | 32.2 | 42.8 |
| GM-CSF | 1.0 | 1.2 | 42.2 | 57.7 | 50.9 | 52.3 |
| MMP3 | 1.0 | 4.5 | 40.0 | 64.9 | 60.5 | 40.3 |
| VEGFA | 1.0 | 0.5 | 4.0 | 6.2 | 5.0 | 3.7 |
| BDNF | 1.0 | 1.2 | 2.0 | 7.5 | 4.4 | 3.7 |
| GDNF | 1.0 | 0.8 | 3.2 | 4.6 | 4.0 | 2.5 |
| NGF | 1.0 | 0.7 | 1.9 | 3.1 | 2.5 | 1.8 |
| NT-3 | 1.0 | 1.0 | 3.0 | 4.2 | 3.8 | 3.1 |

The expression levels of the angiogenesis-inducing factor/neurotrophic factor GM-CSF and MMP3 were higher in the membrane-separated cells than in the unseparated human dental pulp test cells by 10 times or more, regardless of the concentration of G-CSF. The expression levels of VEGF, BDNF, GDNF, NGF and NT-3 in the membrane-separated cells were almost the same levels or 2 times greater than those in CD105⁺ cells (in the case of the membrane-separated cells separated with 100 ng/ml G-CSF), and were higher than those in the unseparated human dental pulp test cells. The expression level of the stem cell marker Sox2 was higher in the membrane-separated cells than in the unseparated cells by 10 times or more. The expression levels of Oct4, Nanog and Rex1 in the membrane-separated cells were almost the same levels or 2 times greater than CD105⁺ cells (in the case of the membrane-separated cells separated with 100 ng/ml G-CSF).

A graph showing a comparison regarding the cell proliferative ability of membrane-separated cells to human serum (* *p* < 0.05) is shown in Fig. 16. In terms of the proliferative ability to serum, no significant difference was found in all of cellular fractions. A comparison regarding the cell proliferative ability of membrane-separated cells, in which G-CSF (100 ng/ml) was used (***p* < 0.01, ** p* < 0.05 : vs unseparated human dental pulp test cells, ^{##} *p* < 0.01 : vs dental pulp CD105⁺ cells), is shown in Fig. 17. The proliferative ability to G-CSF of the membrane-separated cells separated with 100 ng/ml and 500 ng/ml G-CSF was the highest, and thus, the membrane-separated cells had a significant difference from both the unseparated human dental pulp test cells and CD105⁺ cells. A comparison regarding the cell migration ability of membrane-separated cells to G-CSF having different concentrations (***p* < 0.01, ** p* < 0.05 : vs unseparated human dental pulp test cells, ^{##}*p <* 0.01, *^{#}p* < 0.05 : vs dental pulp CD105⁺ cells) is shown in Fig. 18. The migration ability to G-CSF of the membrane-separated cells separated with 100 ng/ml G-CSF was highest, and thus, the membrane-separated cells had a significant difference from both the unseparated human dental pulp test cells and CD105⁺ cells.

### [5. Angiogenesis-inducing ability of membrane-separated cells examined in vivo using mouse lower limb ischemia model]

A mouse lower limb ischemia model was produced, and into its ischemic site, unseparated human dental pulp test cells, membrane-separated cells separated with 100 ng/ml G-CSF, and CD105⁺ cells were each transplanted. Fourteen days later, blood flow was analyzed by laser Doppler technique. With regard to angiogenesis, frozen sections were produced, and were then subjected to immunohistological analysis via BS-1 lectin staining.

As a result of the laser Doppler analysis, it was found that blood flow was not improved so much by transplantation of the test cells, but that blood flow was significantly improved by transplantation of the membrane-separated cells, as in the case of the transplantation of the CD105⁺ cells. Moreover, when frozen sections were produced and were then stained with BS-1 lectin, angiogenesis was observed by transplantation of the membrane-separated cells, as in the case of transplantation of CD105⁺ cells.

### [6. Ability of membrane-separated cells to regenerate dental pulp examined in vivo using SCID mouse]

An extracted human tooth was sliced, and one end thereof was then sealed with cement. Thereafter, unseparated human dental pulp test cells, membrane-separated cells separated with 100 ng/ml G-CSF, or CD105⁺ cells were used as Scaffold, and the cells were injected into the tooth section together with collagen. The resulting tooth section was transplanted into the subcutis of an SCID mouse. Three weeks later, the above three types of cells were compared with one another in terms of ability to regenerate the dental pulp. The morphology was analyzed by HE staining. The nerve regeneration ability and angiogenesis-inducing ability were immunohistologically analyzed by PGP9.5, BS1 lectin staining and Ki67 staining. Localization of the transplanted cells was analyzed by in situ hybridization performed on the human-specific gene Alu. Moreover, the expression level of the dental pulp-specific marker mRNA in the regenerated dental pulp tissues was compared with that in normal dental pulp tissues.

As a result of the HE staining, formation of dental pulp-like tissues was observed by transplantation of the membrane-separated human cells, as in the case of transplantation of CD105⁺ cells. On the other hand, by transplantation of the unseparated test cells, only a low level of formation of dental pulp-like tissues was observed. In addition, as a result of the BS1 lectin and PGP9.5 staining, angiogenesis and regeneration of nerves were observed by transplantation of the membrane-separated cells, as in the case of transplantation of CD105⁺ cells. Moreover, proliferation of the transplanted cells was hardly observed. As a result of the in situ hybridization performed on Alu, it became clear that the host mouse cells form the regenerated dental pulp-like tissues.

The results obtained by examining at an mRNA expression level that the regenerated dental pulp-like tissues are dental pulp are shown in Table 7.

**[Table 7]**

| | mouse normal dental pulp tissue | regenerated dental pulp tissue | |
|---|---|---|---|
| | | unseparated test cells transplantation | membrane-separated cells transplantation |
| Syndecan 3 | 1.0 | 0.7 | 1.5 |
| Tenascin C | 1.0 | 0.2 | 0.7 |
| TRH-DE | 1.0 | 0.7 | 1.8 |
| Periostin | 1.0 | 5.9 | 0.9 |
| aP2 | - | - | - |
| Runx 2 | 1.0 | 0.1 | 0.2 |
| Enamelysin | 1.0 | 0.0 | 0.0 |

The expression level of TRH-DE serving as a dental pulp-specific marker and the expression levels of Syndecan and Tenascin C that are reportedly expressed at high levels in the dental pulp in the regenerated dental pulp-like tissues were the same levels as those in normal dental pulp tissues. On the other hand, the expression of periodontium, adipose tissue, and bone/dentin marker mRNAs was not observed in the regenerated dental pulp-like tissues. From these results, it became clear that the regenerated dental pulp-like tissues obtained by transplantation of the membrane-separated cells are dental pulp.

### Example 6

### [Method for separating dental pulp, bone marrow, and adipose stem cells by membrane]

### [1. Separation of dental pulp, bone marrow, and adipose stem cells using membrane separation device]

Whether stem cells can also be separated from the bone marrow or adipose cells by a membrane separation method, as in the case of dental pulp cells, was examined. As a membrane separation device, Cellculture Insert (polycarbonate base material membrane (Polycarbonate Membrane) Transwell (registered trademark) Inserts; 2 × 10⁵ pores/cm², pore size: 8 µm, diameter of bottom surface: 6.4 mm, diameter of opening portion: 11.0 mm, height: 17.5 mm) (Corning), used an upper structure, was inserted into a 24-well plate (diameter: 15.0 mm, diameter of opening portion: 15.0 mm, height: 22.0 mm) (Falcon), used as a lower structure, and the thus prepared device was used as a membrane separation device. In order to avoid cell adhesion, the surface of the polycarbonate base material membrane was subjected to a coating treatment. In order to impart a non-cell-adhesive property to the polycarbonate base material, the surface of the polycarbonate base material membrane had previously been modified by immersing it in a treating aqueous solution prepared by adding 0.1% ethanol to a 1000 ppm aqueous solution of a polyvinyl pyrrolidone-polyvinyl acetate copolymer (vinyl pyrrolidone/vinyl acetate (6/4) copolymer ("Kollidon VA64," manufactured by BASF)) so as to seal it, irradiating the resulting membrane with a γ-ray (25 kGy), thereby preparing a separation membrane. Thereafter, the pig dental pulp, bone marrow, and adipose cells at the 2nd passage of culture were each dispersed on the upper portion of this separation membrane at a cell density of 1.5 × 10⁴ cells/100 µl. Meanwhile, G-CSF (final concentration: 100 ng/ml) was added into Dulbecco's modified Eagle's medium (DMEM) containing 10% FBS in 24 wells of the lower structure of the membrane. Twenty-four hours later, the G-CSF was removed, and the medium was replaced with another DMEM containing 10% FBS, followed by performing a culture. After the cells had become 70% confluent, they were subcultured.

As a result, it was found that bone marrow-derived and adipose stem cells are also separated in the lower portion of the separation membrane under the same conditions as those applied to separation of the dental pulp stem cells.

### [2. Measurement of positive expression rates of stem cell surface antigen markers]

Membrane-separated pig dental pulp, bone marrow, and adipose cells were subcultured for five generations, and the positive expression rates of stem cell surface antigen markers were then measured. Thereafter, the cells were dispersed in PBS containing 2% FBS at a cell density of 1 × 10⁶ cells/ml. After that, the cells were labeled with stem cell marker antibodies at 4°C for 60 minutes, followed by performing flow cytometry. Specifically, the cells were labeled at 4°C for 60 minutes using mouse IgG1 negative control (AbD Serotec Ltd.), hamster IgG (PE-cy7) (eBio299Arm) (eBioscience), rat IgG2b (PE-cy7) (RTK4530) (Biolegend), mouse IgG1 (APC) (NOPC-21) (Biolegend), mouse IgG1 (RPE) (SFL928PE) (AbD Serotec), mouse IgG1 (Alexa647) (F8-11-13) (AbD Serotec), mouse IgG2a (FITC) (S43.10) (MACS), mouse IgG1 (FITC) (MOPC-21) (Biolegend), antibodies to the following: CD29 (Phycoerythrin, PE-cy7) (eBioHMb1-1) (eBioscience), CD31 (PE) (LCI-4) (AbD Serotec), CD44 (PE-cy7) (IM7) (eBioscience), CD73 (APC) (AD2) (Biolegend), CD90 (Alexa647) (F15-42-1) (AbD Serotec), CD105 (FITC) (MEM-229) (Abcam), CXCR4 (FITC) (12G5) (R&D Systems), G-CSFR (CD114) (Alexa 488) (S1390) (Abcam). After completion of the labeling, Hank's buffer, to which Hepes had been added to a final concentration of 0.01 M and FBS had also been added to a final concentration of 2%, was added to the resulting cells, followed by performing flow cytometry. As negative controls, unseparated dental pulp, bone marrow, and adipose test cells were used.

The comparative results regarding the expression rates of the stem cell markers, obtained by flow cytometry, are shown in Table 8.

**[Table 8]**

| | dental pulp | | bone marrow | | adipose | |
|---|---|---|---|---|---|---|
| | unseparated test cells | membrane-separated cells | unseparated test cells | membrane-separated cells | unseparated test cells | membrane-separated cells |
| CD29 | 99.5 % | 96.7% | 93.6 % | 94.7 % | 94.3 % | 92.4 % |
| CD44 | 97.2 % | 96.0% | 92.8 % | 93.7 % | 99.2 % | 97.3 % |
| CD73 | 92.6 % | 91.0% | 90.6 % | 91.7 % | 90.1 % | 90.1 % |
| CD90 | 94.8 % | 92.4% | 90.3 % | 90.9 % | 90.7 % | 90.4 % |
| CD105 | 14.7 % | 70.8% | 22.3 % | 73.2 % | 25.9 % | 61.7 % |
| CXCR4 | 5.9 % | 14.1 % | 4.1 % | 14.2 % | 2.8 % | 7.0 % |
| G-CSFR | 23.7 % | 74.2 % | 21.9 % | 48.5 % | 23.6 % | 49.5 % |

As a result, CD29, CD44, CD73, and CD90 were almost positively expressed, and there were found no differences between the membrane-separated cells and the unseparated test cells. The positive expression rates of CD105 in unseparated dental pulp, bone marrow, and adipose test cells were 14.7%, 22.3%, and 25.9%, respectively. On the other hand, the positive expression rates of CD105 in the membrane-separated dental pulp, bone marrow, and adipose cells were 70.8%, 73.2%, and 61.7%, respectively. Thus, the positive expression rate of CD105 was higher in the membrane-separated cells than in the unseparated test cells, regardless of the types of the cells. Moreover, the positive expression rates of CXCR4 in unseparated dental pulp, bone marrow, and adipose test cells were 5.9%, 4.1%, and 2.8%, respectively. On the other hand, the positive expression rates of CXCR4 in the membrane-separated dental pulp, bone marrow, and adipose cells were 14.1%, 14.2%, and 7.0%, respectively, thereby showing higher positive expression rates. Furthermore, in the case of G-CSFR as a G-CSF receptor as well, the positive expression rates of G-CSFR in unseparated dental pulp, bone marrow, and adipose test cells were 23.7%, 21.9%, and 23.6%, respectively, whereas the positive expression rates of G-CSFR in the membrane-separated dental pulp, bone marrow, and adipose cells were 74.2%, 48.5%, and 49.5%, respectively, showing higher positive expression rates. From these results, it was found that stem cells/precursor cells, in which the positive expression rates of CD105, CXCR4 and G-CSFR are high, can be separated according to a membrane separation method, not only from dental pulp cells, but also from bone marrow cells and adipose cells.

### [3. Analysis of expression of mRNAs of angiogenic factors, neurotrophic factors, and stem cell markers]

Subsequently, the expression of the mRNAs of angiogenic factors, neurotrophic factors, and stem cell markers was analyzed by real-time RT-PCR. Using Trizol (Invitrogen), total RNA was extracted from each of the membrane-separated dental pulp, bone marrow and adipose cells, which had been subcultured for 5 generations, and also from each of the unseparated dental pulp, bone marrow and adipose cells, which had been subcultured for 5 generations. The thus extracted total RNA was treated with DNase (Roche), and thereafter, first-strand cDNA was synthesized therefrom using ReverTra Ace-α (TOYOBO). The synthesized cDNA was labeled with Light Cycler-Fast Start DNA master SYBR Green I (Roche Diagnostics). Thereafter, real-time RT- PCR was performed for angiogenic factors, neurotrophic factors, and stem cell markers, employing Light Cycler (Roche Diagnostics) in accordance with a program of 95°C-10 seconds, 65°C or 60°C-15 seconds, and 72°C-8 seconds. Primers used as such angiogenic factors, neurotrophic factors, and stem cell markers are shown in Table 9. The primers of granulocyte-monocyte colony-stimulating factor (GM-CSF), vascula endothelin growth factor (VEGF)-A, matrix metalloproteinase (MMP)-3, chemokine (C-X-C motif) receptor (CXCR)-4, brain-derived neurotrophic factor (BDNF), glial cell line-derived neurotrophic factor (GDNF), nerve growth factor (NGF), nanog homeobox (Nanog), SRY (sex determining region Y)-box 2 (Sox2), signal transducer and activator of transcription (STAT)-3, telomerase reverse transcriptase (Tert), Bmi1 polycomb ring finger oncogene (Bmi-1) were standardized with β-actin.

**[Table 9]**

| Gene | | 5'←DNA Sequence→3' | product size | Accession number |
|---|---|---|---|---|
| Nanog | Forward | CCCCGAAGCATCCATTTCC(SEQ ID NO. 51) | 101 bp | DQ447201 |
| | Reverse | CGAGGGTCTCAGCAGATGACAT(SEQ ID NO. 52) | | |
| Sox2 | Forward | AATGCCTTCATGGTGTGGTC(SEQ ID NO. 53) | 203 bp | DQ400923 |
| | Reverse | CGGGGCCGGTATTTATAATC(SEQ ID NO. 54) | | |
| STAT3 | Forward | GTGGTGACAGAGAAGCAGCA(SEQ ID NO. 55) | 191 bp | NM_001044580 |
| | Reverse | TTCTGCCTGGTCACTGACTG(SEQ ID NO. 56) | | |
| Tert | Forward | CAGGTGTACCGCCTCCTG(SEQ ID NO. 57) | 180 bp | DQ400924 |
| | Reverse | CCAGATGCAGTCTTGCACTT(SEQ ID NO. 58) | | |
| Bmi-1 | Forward | ATATTTACGGTGCCCAGCAG(SEQ ID NO. 59) | 179 bp | |
| | Reverse | GAAGTGGCCCATTCCTTCTC(SEQ ID NO. 60) | | |
| CXCR4 | Forward | CCGTGGCAAACTGGTACTTT(SEQ ID NO. 61) | 209 bp | NM_213773 |
| | Reverse | TCAACAGGAGGGCAGGTATC(SEQ ID NO. 62) | | |
| GM-CSF | Forward | GCCCTGAGCCTTCTAAACAA(SEQ ID NO. 63) | 193 bp | AY116504 |
| | Reverse | GTGCTGCTCATAGTGCTTGG(SEQ ID NO. 64) | | |
| MMP3 | Forward | ACCCAGATGTGGAGTTCCTG(SEQ ID NO. 65) | 171 bp | NM_001166308 |
| | Reverse | GGAGTCACTTCCTCCCAGATT(SEQ ID NO. 66) | | |
| VEGFA | Forward | ATGGCAGAAGGAGACCAGAA(SEQ ID NO. 67) | 224 bp | NM_214084 |
| | Reverse | ATGGCGATGTTGAACTCCTC(SEQ ID NO. 68) | | |
| BDNF | Forward | TTCAAGAGGCCTGACATCGT(SEQ ID NO. 69) | 180 bp | NM_214259 |
| | Reverse | AGAAGAGGAGGCTCCAAAGG(SEQ ID NO. 70) | | |
| GDNF | Forward | ACGGCCATACACCTCAATGT(SEQ ID NO. 71) | 111 bp | XM_003133897 |
| | Reverse | CCGTCTGTTTTTGGACAGGT(SEQ ID NO. 72) | | |
| NGF | Forward | TGGTGTTGGGAGAGGTGAAT(SEQ ID NO. 73) | 210 bp | XM_003355233 |
| | Reverse | CCGTGTCGATTCGGATAAA(SEQ ID NO. 74) | | |
| *β* -actin | Forward | CTCTTCCAGCCCTCCTTCCT(SEQ ID NO. 75) | 80 bp | AJ312193 |
| | Reverse | ACGTCGCACTTCATGATCGA(SEQ ID NO. 76) | | |

The results obtained by analyzing the expression of mRNAs of the angiogenesis-inducing factors and the neurotrophic factors are shown in Table 10. The expression levels of GM-CSF, MMP3, and BDNF were higher in the membrane-separated cells than in the unseparated test cells by approximately 5 to 10 times. On the other hand, the expression levels of VEGF, GDNF, and NGF in the membrane-separated cells were almost the same levels or 2 times greater than those in CD105⁺ cells (in the case of the membrane-separated cells separated with 100 ng/ml G-CSF), and were higher in the membrane-separated cells than in the unseparated dental pulp test cells.

**[Table 10]**

| | dental pulp | | bone marrow | | adipose | |
|---|---|---|---|---|---|---|
| | unseparated test cells | membrane-separated cells | unseparated test cells | membrane-separated cells | unseparated test cells | membrane-separated cells |
| Nanog | 1.0 | 2.3 | 0.3 | 1.2 | 0.3 | 0.5 |
| Sox2 | 1.0 | 17.3 | 0.5 | 1.4 | 0.2 | 0.4 |
| STAT3 | 1.0 | 2.2 | 0.7 | 1.3 | 0.4 | 0.5 |
| GM-CSF | 1.0 | 6.3 | 0.7 | 3.9 | 0.3 | 0.4 |
| VEGF | 1.0 | 2.1 | 0.4 | 0.5 | 0.2 | 0.4 |
| MMP3 | 1.0 | 10.9 | 0.6 | 2.1 | 0.4 | 0.7 |
| CXCR4 | 1.0 | 3.8 | 1.0 | 2.5 | 1.0 | 2.2 |
| BDNF | 1.0 | 6.3 | 0.6 | 3.3 | 2.0 | 4.4 |
| GDNF | 1.0 | 5.7 | 1.3 | 2.8 | 0.9 | 1.0 |
| NGF | 1.0 | 5.3 | 0.6 | 2.0 | 0.9 | 1.2 |

### [4. Analysis of angiogenesis-inducing ability, cell proliferative ability, and cell migration ability]

The angiogenesis-inducing ability, cell proliferative ability, cell migration ability of the 5^{th}-generation dental pulp, bone marrow, and adipose membrane-separated cells were examined *in vitro.* With regard to angiogenesis-inducing ability, the aforementioned different types of cells dispersed on an EGM-2 (Lonza) medium were each subjected to a three-dimensional culture on matrigel, and the cultured cells were compared regarding lumen formation ability. With regard to cell proliferative ability, using TetraColor One (Seikagaku Biobusiness Corporation), cell proliferative ability was measured by stimulation with 10% FBS or 100 ng/ml G-CSF. Moreover, cell migration ability to G-CSF was analyzed by real-time horizontal chemotaxis analysis using TAXIScan-FL. Specifically, a channel optimized to the size of cells (8 µm) was formed between silicone having pores with a pore size of 6 µm and a glass plate. Thereafter, membrane-separated pig dental pulp, bone marrow and adipose cells, and unseparated pig dental pulp, bone marrow and adipose test cells, were each poured in one side of the channel (1 µl each; cell density: 10⁵ cells/ml). Various types of migration factors (10 ng/µl) were each poured into the opposite side thereof, so as to form a certain concentration gradient. Based on video images of migration, the number of migrating cells was counted every 30 minutes until 24 hours after initiation of the operation.

With regard to angiogenesis induction, all of the membrane-separated dental pulp, bone marrow, and adipose-derived cells were observed to form a cord-like structure on matrigel 5 hours after initiation of the operation, thereby exhibiting ability to differentiate into vascular endothelial cells. On the other hand, the unseparated test cells did not form such a cord-like structure even after observation for a long period of time.

A graph regarding a comparison of cell proliferative ability in which fetal bovine serum was used is shown in Fig. 19, and a graph regarding a comparison of cell proliferative ability in which G-CSF was used is shown in Fig. 20. The cell proliferative ability of the membrane-separated cells examined using FBS and G-CSF was higher than that of the unseparated test cells. A graph regarding the measurement of the number of migrating cells using G-CSF is shown in Fig. 21. It was found that the cell migration ability of the membrane-separated cells examined using G-CSF was highest among all types of unseparated test cells.

### Example 7

### [Method for directly separating stem cells from fresh dental pulp and adipose tissues by membrane]

Whether stem cells can be directly separated from dental pulp and adipose tissues by a membrane separation method without performing enzyme digestion, as with other cells, was examined. As a membrane separation device, Cellculture Insert (a surface-modified polycarbonate base material membrane Transwell (registered trademark) Inserts; 2 × 10⁵ pores/cm², pore size: 8 µm, diameter of bottom surface: 6.4 mm, diameter of opening portion: 11.0 mm, height: 17.5 mm) (Corning), used an upper structure, was inserted into a 24-well plate (diameter: 15.0 mm, diameter of opening portion: 15.0 mm, height: 22.0 mm) (Falcon), used as a lower structure, and the thus prepared device was used as a membrane separation device. As in the case of Example 6 as described above, this polycarbonate base material membrane had also been subjected to a treatment for imparting a non-cell-adhesive property thereto, and a separation membrane was then prepared. The prepared separation membrane was incorporated into the membrane separation device. Thereafter, minced fresh dog dental pulp tissues or adipose tissues were left at rest on the upper portion of this polycarbonate membrane, meanwhile, G-CSF (final concentration: 100 ng/ml) was added into Dulbecco's modified Eagle's medium (DMEM) containing 10% FBS in 24 wells of the lower structure of the membrane. Twenty-four hours later, the G-CSF was removed, and the medium was replaced with another DMEM containing 10% FBS, followed by performing a culture. After the cells had become 70% confluent, they were subcultured.

The states of dental pulp stem cells and adipose stem cells that have migrated and adhered 24 hours after initiation of the operation are shown in Fig. 23. As a result, it was found that the cells could also be separated also from dental pulp tissues and adipose tissues under the same conditions as those for separation from the test cells and were collected in the lower portion of the membrane.

### Example 8

Next, the present invention will be described in the following examples and comparative examples regarding the separation membrane used in the present invention.

### [1. Measurement methods]

### (1) Electron Spectroscopy for Chemical Analysis (ESCA) Measurement

Three points on each of the inner surface and outer surface of a separation membrane were measured. The measurement sample was rinsed with ultrapure water, was then dried at a room temperature at 66.7 Pa (0.5 Torr) for 10 hours, and was then subjected to measurement. The measurement device and measurement conditions are the following:
Measurement device: ESCLAB220iXL
Excitation X-ray: monochromatic AlKa 1,2 ray (1486.6 eV)
X-ray diameter: 0.15 mm
Photoelectron escape angle: 90° (inclination of detector to sample surface)

Moreover, by analyzing the separation membrane used in the present invention by an elementary analysis method, the amount of a hydrophilic polymer on the surface of the separation membrane can be calculated, for example, based on the amount of nitrogen (a (atom number %)) and the amount of sulfur (b (atom number %)), etc. In the case of polyacrylonitrile, a calibration curve of film was prepared based on the ratio of the peak strength of C≡N derived from nitrile groups around 2,200 cm⁻¹ (ACN) and ACO in the same manner as described above, and the ratio of an internal vinyl acetate unit amount was then obtained.

### (2) Method for measuring hydrophilic polymer distribution according to infrared absorption spectrometry

The separation membrane was rinsed with ultrapure water, and was then dried at a room temperature at 66.7 Pa (0.5 Torr) for 10 hours. The surface of the thus dried separation membrane was measured by microscopic ATR method using IRT-3000 manufactured by JASCO. A visual field region (aperture) was set at 100 µm × 100 µm, the cumulated number per single point was set at 30, and the aperture was moved by each 3 µm, so that the measurement was carried out at a total of 25 points consisting of 5 points in the longitudinal direction and 5 points in the vertical direction. Moreover, based on the measurement of a difference spectrum from a surface-not-modified membrane, the amount of the adhered hydrophilic polymer was calculated.

### (3) Method for testing adhesion of human platelets to membrane

A separation membrane was attached to a Falcon (registered trademark) tube (18 mmϕ, No. 2051) cut into a cylindrical shape, such that the surface of the membrane to be evaluated could be inside the cylinder. Then, the space was filled with paraffin. The inside of this cylindrical tube was washed with a normal saline, and was then filled with a normal saline. Venous blood was collected from a healthy volunteer, and heparin was immediately added to the collected blood to a concentration of 50 U/ml. The normal saline was discarded from the cylindrical tube. Thereafter, 1.0 ml of the blood was added into the cylindrical tube within 10 minutes after blood collection, and it was then shaken at 37°C for 1 hour. Subsequently, a hollow fiber membrane was washed with 10 ml of a normal saline, blood component was then immobilized thereon with a 2.5 weight % glutaraldehyde normal saline, and the membrane was then washed with 20 ml of distilled water. The washed separation membrane was dried under a reduced pressure at an ordinary temperature at 66.7 Pa (0.5 Torr) for 10 hours. Thereafter, using a double stick tape, the resulting separation membrane was attached on a sample stand of a scanning electron microscope. After that, a thin membrane of Pt-Pd was formed on the surface of the hollow fiber membrane by sputtering, so as to prepare a sample. The inner surface of this separation membrane was observed at a magnification of 1,500 times under field emission scanning electron microscope (S800, manufactured by Hitachi), and the number of platelets adhered to a single visual field (4.3 × 10³ µm²) was counted. A mean value of platelets adhered to 10 different visual fields around the center in the longitudinal direction of the hollow fiber was defined as the number of adhering platelets (platelets/4.3 × 10³ µm²).

The number of platelets adhered to a material having a good platelet adhesion-suppressing property is 40 or less (platelets/4.3 × 10³ µm²), preferably 20 or less (platelets/4.3 × 10³ µm²), and more preferably 10 or less 0(platelets/4.3 × 10³ µm²) or less.

### (4) Evaluation of cell permeation

The separation membrane used in the present invention attached to Cellculture Insert manufactured by BD was used, and "Mesenchymal Stem Cell" manufactured by PromoCell was used herein as mesenchymal stem cells. Mesenchymal Stem Cell Adipogenic Differentiation Medium #C-28011, Ready-to-use (a medium used for proliferation of mesenchymal stem cells) was used as a medium for differentiation and culture, and this medium was placed in the lower portion of the above-modified Cellculture Insert manufactured by BD. On the other hand, the above-mentioned cells and a medium for culture (Mesenchymal Stem Cell Expansion Media, Human/Mouse, StemXVivo) were placed in the upper portion of the modified Cellculture Insert. Moreover, G-CSF was further added into the medium in the lower portion to a final concentration of 100 ng/ml, and it was then left at 37°C in a CO₂ incubator for 12 hours. The number of cells dropped from the upper portion to the lower petri dish for 12 hours was counted under a phase contrast microscope.

The water absorption percentage of each of a PET membrane, a polycarbonate membrane, a PP membrane and a polysulfone membrane was 2% or less, although the water absorption percentage of a polyamide membrane was 4.2%. The used base material membrane was immersed in water at 23°C for 24 hours, and an increased weight was defined as the water absorption percentage of a polymer.

In accordance with such water absorption percentage, using a membrane consisting of polycarbonate and a polyamide membrane ("Nylon 66"), a surface treatment was carried out under conditions as described in the table below. A membrane portion of the "Cellculture Insert" was appropriately replaced with the used membrane, and cells were then allowed to migrate using a G-CSF migration factor. Thereafter, the number of cells that permeated through the membrane and adhered to the lower petri dish was then counted. It was found that, in the case of using a membrane consisting of PET, the adhesive property of the cells was suppressed in an ethanol (EtOH) addition system, and that a large number of cells dropped and adhered to the lower petri dish. Also, it was found that, in the case of using a polyamide membrane, a cell adhesion-suppressing property was expressed in an EtOH-non-addition system.

The amount of a hydrophilic polymer bound to the surface was appropriately analyzed and was obtained by an elementary analysis method, ESCA, and an IR method. The following hydrophilic polymers were used.
PVP: polyvinyl pyrrolidone (K90, K30), Tokyo Chemical Industry Co., Ltd.
PVA: polyvinyl alcohol (manufactured by Kuraray Co., Ltd.)
VA64: polyvinyl pyrrolidone/polyvinyl acetate copolymer (Kollidon VA64, manufactured by BASF)

### [Results of treatment of polycarbonate membrane]

Cellculture Insert comprising a polycarbonate membrane having pores with a pore diameter of 8 µm was used, and the surface of the membrane was modified by immersing it in aqueous solutions having different conditions, sealing it, and then irradiating it with a γ-ray (25 kGy). Thereafter, human mesenchymal stem cells were dispersed on the upper portion of the membrane (1 × 10⁴ cells/100 µl), and the number of cells adhered to the lower portions of 24 wells was then measured under phase contrast microscope, thereby evaluating separation performance. The results are shown in the following Table 11.

**[Table 11]**

| surface modification | the amount of a hydrophilic polymer adhered (wt%) | the number of cells that permeated through the membrane |
|---|---|---|
| untreated | 0 | 2 |
| VA64 1000ppm+ EtOH 0.1wt% | 31 | 450 |
| VA64 1000ppm | 1.5 | 210 |
| VA64 10ppm+EtOH 0.1wt% | 1.6 | 190 |
| VA64 1000ppm+ EtOH 0.2wt% | 35 | 500 |
| VA64 1000ppm+EtOH 0.01wt% | 11 | 330 |
| PVP(K90)1000ppm+ EtOH 0.1wt% | 16 | 350 |
| PVP(K90) 100ppm+ EtOH 0.01wt% | 1.1 | 35 |

### [Results of treatment of polyamide membrane]

The membrane portion of the Cellculture Insert was replaced with a polyamide membrane having pores with a pore diameter of 8 µm, and the surface of the replaced membrane was modified by immersing it in aqueous solutions having different conditions, sealing it, and then irradiating it with a γ-ray (25 kGy). Thereafter, human mesenchymal stem cells were dispersed on the upper portion of the membrane (1 × 10⁴ cells/100 µl), and the number of cells adhered to the lower portions of 24 wells was then measured under phase contrast microscope, thereby evaluating separation performance. The results are shown in the following Table 12.

**[Table 12]**

| surface modification | amount of a hydrophilic polymer adhered (wt%) | the number of cells that permeated through the membrane |
|---|---|---|
| untreated | 0 | 40 |
| VA64 1000ppm+ EtOH 0.1wt% | 16 | 320 |
| VA64 1000ppm | 33 | 480 |
| VA64 10ppm | 2.8 | 192 |
| VA64 100ppm | 15 | 346 |
| VA64 100ppm+ EtOH 0.1wt% | 11 | 311 |
| PVP(K90) 1000ppm | 18 | 352 |
| PVP(K90) 100ppm+ EtOH 0.01wt% | 1.3 | 56 |

### [Influence of hydrophilic polymer]

Cellculture Insert comprising a polycarbonate membrane having pores with a pore diameter of 8 µm was used, and the surface of the membrane was modified by immersing it in aqueous solutions having different conditions, sealing it, and then irradiating it with a γ-ray (25 kGy). Thereafter, human mesenchymal stem cells were dispersed on the upper portion of the membrane (1 × 10⁴ cells/100 µl), and the number of cells adhered to the lower portions of 24 wells was then measured under phase contrast microscope, thereby evaluating separation performance. The results are shown in the following Table 13.

**[Table 13]**

| surface modification | amount of a hydrophilic polymer adhered (wt%) | the number of cells that permeated through the membrane |
|---|---|---|
| untreated | 0 | 13 |
| PVP K90 1000ppm + EtOH 0.1wt% | 18 | 311 |
| PVP K90 1000ppm | 2.2 | 149 |
| PVP K30 1000ppm + EtOH 0.1wt% | 13 | 255 |
| PVP K30 1% + EtOH 0.1wt% | 16 | 321 |
| PVA417 1000ppm + EtOH 0.1wt% | 17 | 330 |
| PVA417 1000ppm | 1.8 | 116 |
| PEG20,000 1000ppm + EtOH 0.1wt% | 4 | 123 |
| VA64 1000ppm+ EtOH 0.1wt% | 33 | 436 |
| VA64 1000ppm | 1.9 | 132 |
| PVP(K90) 100ppm+ EtOH 0.01wt% | 1.3 | 43 |

### [Confirmation of platelet adhesive property]

Using a film consisting of PET and having no pores, the number of platelets adhered thereto was counted under various conditions. The results are shown in the following Table 14.

**[Table 14]**

| surface modification | the amount of a hydrophilic polymer adhered (wt%) | the number of platelets adhered/4.3 × 10³ *µ*m² |
|---|---|---|
| untreated | 0 | 230 |
| PVP K90 1000ppm + EtOH 0.1wt% | 20 | 9 |
| PVP K90 1000ppm | 3.1 | 37 |
| PVP K30 1000ppm + EtOH 0.1wt% | 15 | 22 |
| PVP K30 1% + EtOH 0.1wt% | 16 | 19 |
| PVA417 1000ppm + EtOH 0.1wt% | 15 | 18 |
| PVA417 1000ppm | 2.2 | 33 |
| PEG20,000 1000ppm + EtOH 0.1wt% | 4 | 19 |
| VA64 1000ppm+ EtOH 0.1wt% | 29 | 3 |
| VA64 1000ppm | 22 | 31 |
| PVP(K90) 100ppm+ EtOH 0.01wt% | 1.2 | 157 |

### [Results of treatment of polycarbonate membrane]

Cellculture Insert comprising a polycarbonate membrane having pores with a pore diameter of 5 µm was used, and the surface of the membrane was modified by immersing it in aqueous solutions having different conditions, sealing it, and then irradiating it with a γ-ray (25 kGy). Thereafter, human dental pulp cells at the 2nd passage of culture were dispersed on the upper portion of the membrane (2 × 10⁴ cells/100 µl), and G-CSF (final concentration: 100 ng/ml) was added into Dulbecco's modified Eagle's medium (DMEM) containing 10% human serum in the 24 wells of the lower structure thereof. Forty-eight hours later, the G-CSF was removed, and the medium was replaced with another DMEM containing 10% human serum. Thereafter, the number of cells adhered to the lower portion of the 24 wells was counted under phase contrast microscope. The results are shown in Table 15.

**[Table 15]**

| surface modification | amount of a hydrophilic polymer adhered (wt%) | cell permeation rate (%) |
|---|---|---|
| untreated | 0 | 0.2 |
| VA64 1000ppm+ EtOH 0.1wt% | 28 | 4.5 |
| VA64 1000ppm | 1.7 | 2.1 |
| VA64 10ppm+EtOH 0.1wt% | 1.9 | 1.9 |
| VA64 1000ppm+ EtOH 0.2wt% | 31 | 4.4 |
| VA64 1000ppm+EtOH 0.01wt% | 14 | 3.3 |
| PVP(K90)1000ppm+ EtOH 0.1wt% | 17 | 6.5 |
| PVP(K90) 100ppm+ EtOH 0.01wt% | 1.3 | 0.3 |

### [Results of treatment of polyamide membrane]

Subsequently, the membrane portion of the Cellculture Insert was replaced with a polyamide membrane having pores with a pore diameter of 5 µm, and the surface of the replaced membrane was modified by immersing it in aqueous solutions having different conditions, sealing it, and then irradiating it with a γ-ray (25 kGy). Thereafter, the same operations as those described above were carried out. The obtained results are shown in Table 16.

**[Table 16]**

| surface modification | amount of a hydrophilic polymer adhered (wt%) | cell permeation rate (%) |
|---|---|---|
| untreated | 0 | 0.4 |
| VA64 1000ppm+ EtOH 0.1wt% | 15 | 3.2 |
| VA64 1000ppm | 31 | 4.8 |
| VA64 10ppm | 2.2 | 1.9 |
| VA64 100ppm | 16 | 3.5 |
| VA64 100ppm+ EtOH 0.1wt% | 12 | 3.1 |
| PVP(K90) 1000ppm | 21 | 3.5 |
| PVP(K90) 100ppm+ EtOH 0.01wt% | 1.2 | 0.5 |

### Reference Signs List

1, 2, 3, 4,5 Membrane separation culture device
10, 20, 30, 40, 50, 60 Upper structure
12, 22, 32, 62 Separation membrane
121, 221 Pore
13, 23, 33, 43, 53, 63 Lower structure
231 Medium inlet port
232 Medium outlet port
24, 34, 44 Lid structure
241 Gas inlet port
242 Gas discharge port
331, 431 Elastic body
35, 335 Retention mechanism
45, 55 Frame body
451, 551 Hole
452, 552 Partition
453, 553 Retention mechanism
64 Lid structure
65 Introduction port
66 Dish
67 Lid structure
661 Medium recovery port
100 Medium
200 Test cells
300 Medium
a Inert gas
b Emission gas
c Medium
d Used medium

## Claims

1. A membrane separation culture device comprising:
an upper structure constituted with a vessel in which at least a portion of the bottom thereof is formed with a separation membrane having pores that allow stem cells to permeate therethrough; and
a lower structure constituted with a vessel that retains a fluid in which the separation membrane of the upper structure is immersed,
wherein
the separation membrane comprises:
a base material membrane consisting of a hydrophobic polymer; and
a functional layer formed by allowing one or more hydrophilic polymers selected from a vinyl pyrrolidone polymer, a polyethylene glycol polymer and a vinyl alcohol polymer to bind to the surface of the base material membrane via a covalent bond; wherein
the weight percentage of the hydrophilic polymer(s) constituting the functional layer is 1.5% to 35% based on the total weight of the separation membrane, and
wherein the size of the pore is 3 µm to 10 µm and the pore density is 1 × 10⁵ to 4 × 10⁶ pores/cm².

2. The membrane separation culture device according to claim 1, wherein the plurality of the upper structures have separation membranes each having a different pore size and/or a different pore density.

3. A membrane separation culture kit comprising a membrane separation culture device according to any one of claims 1 and 2 and cell migration factor(s) to be poured into the lower structure,
wherein the cell migration factor(s) are one or more selected from SDF-1, G-CSF, bFGF, TGF-β, NGF, PDGF, BDNF, GDNF, EGF, VEGF, MMP3, Slit, GM-CSF, LIF, HGF, and serum.

4. The kit according to claim 3, wherein the concentration of the cell migration factor(s) is 1 ng/ml to 500 ng/ml.

5. The kit according to claim 3, which further comprises serum to be poured into the lower structure and wherein the cell migration factor is G-CSF or bFGF.

6. A method for separating stem cells using the membrane separation culture device according to any one of claims 1 and 2, wherein the method comprises:
a step of dispersing test cells or test tissues on the separation membrane of the upper structure;
a step of filling the vessel as a lower structure with a medium containing cell migration factor(s); and
a step of allowing the separation membrane of the upper structure to come into contact with the medium in the lower structure,
wherein the cell migration factor(s) are one or more selected from SDF-1, G-CSF, bFGF, TGF-β, NGF, PDGF, BDNF, GDNF, EGF, VEGF, MMP3, Slit, GM-CSF, LIF, HGF, and serum.

7. The method according to claim 6, wherein the concentration of the cell migration factor(s) is 1 ng/ml to 500 ng/ml.

## Patentansprüche

1. Membrantrennkultur-Vorrichtung, umfassend:
einen oberen Aufbau, der mit einem Gefäß versehen ist, in dem wenigstens ein Teil des Bodens durch eine Trennmembrane gebildet wird, die Poren aufweist, die ein Hindurchgehen von Stammzellen erlauben, und
einen unteren Aufbau, der mit einem Gefäß versehen ist, das ein Fluid hält, in das die Trennmembrane des oberen Aufbaus eingetaucht wird,
wobei die Trennmembrane umfasst:
eine Basismaterial-Membrane, die aus einem hydrophoben Polymer besteht, und
eine Funktionsschicht, die ausgebildet wird, indem erlaubt wird, dass sich ein oder mehrere hydrophile Polymere, die aus einem Vinylpyrrolidon-Polymer, einem Polyethylenglycol-Polymer und einem Vinylalkohol-Polymer ausgewählt sind, an die Oberfläche der Basismaterial-Membrane mittels einer kovalenten Bindung binden,
wobei der Gewichtsprozentsatz des oder der hydrophilen Polymere, das bzw. die die Funktionsschicht bilden, 1,5% bis 35% basierend auf dem Gesamtgewicht der Trennmembrane beträgt, und
wobei die Porengröße 3 µm bis 10 µm beträgt und die Porendichte 1 × 10⁵ bis 4 × 10⁶ Poren/cm² beträgt.

2. Membrantrennkultur-Vorrichtung nach Anspruch 1, wobei die Vielzahl von oberen Aufbauten Trennmembranen aufweisen, die jeweils eine andere Porengröße und/oder eine andere Porendichte aufweisen.

3. Membrantrennkultur-Satz, der eine Membrantrennkultur-Vorrichtung gemäß Anspruch 1 oder 2 und einen oder mehrere in den unteren Aufbau zu gießende Zellmigrationsfaktoren umfasst,
wobei der oder die Zellemigrationsfaktoren einer oder mehrere sind, die aus SDF-1, G-CSF, bFGF, TGF-β, NGF, PDGF, BDNF, GDNF, EGF, VEGF, MMP3, Slit, GM-CSF, LIF, HGF und Serum ausgewählt werden.

4. Satz nach Anspruch 3, wobei die Konzentration des oder der Zellmigrationsfaktoren 1 ng/ml bis 500 ng/ml beträgt.

5. Satz nach Anspruch 3, der weiterhin ein in den unteren Aufbau zu gießendes Serum umfasst und wobei der Zellmigrationsfaktor G-CSF oder bFGF ist.

6. Verfahren zum Trennen von Stammzellen unter Verwendung der Membrantrennkultur-Vorrichtung gemäß Anspruch 1 oder 2, wobei das Verfahren umfasst:
einen Schritt zum Verteilen von Testzellen oder Testgeweben auf der Trennmembrane des oberen Aufbaus,
einen Schritt zum Füllen des Gefäßes als eines unteren Aufbaus mit einem Medium, das einen oder mehrere Zellmigrationsfaktoren enthält, und
einen Schritt zum Erlauben, dass die Trennmembrane des oberen Aufbaus in einen Kontakt mit dem Medium in dem unteren Aufbau kommt,
wobei der oder die Zellemigrationsfaktoren einer oder mehrere sind, die aus SDF-1, G-CSF, bFGF, TGF-β, NGF, PDGF, BDNF, GDNF, EGF, VEGF, MMP3, Slit, GM-CSF, LIF, HGF und Serum ausgewählt werden.

7. Verfahren nach Anspruch 6, wobei die Konzentration des oder der Zellmigrationsfaktoren 1 ng/ml bis 500 ng/ml beträgt.

## Revendications

1. Dispositif de culture par séparation sur membrane comprenant :
une structure supérieure constituée avec un récipient dans lequel au moins une partie du fond de celle-ci est formée avec une membrane de séparation ayant des pores qui permettent à des cellules souches de s'infiltrer à travers ceux-ci ; et
une structure inférieure constituée d'un récipient qui retient un fluide dans lequel la membrane de séparation de la structure supérieure est plongée,
dans lequel
la membrane de séparation comprend :
une membrane en matériau de base constituée d'un polymère hydrophobe ; et
une couche fonctionnelle formée en laissant un ou plusieurs polymères hydrophiles choisis parmi un polymère de vinylpyrrolidone, un polymère de polyéthylène glycol et un polymère d'alcool vinylique de se lier à la surface de la membrane en matériau de base par l'intermédiaire d'une liaison covalente ; dans lequel
le pourcentage en poids du ou des polymères hydrophiles constituant la couche fonctionnelle est de 1,5 % à 35 % par rapport au poids total de la membrane de séparation, et
dans lequel la taille de pore est de 3 µm à 10 µm et la densité des pores est de 1 x 10⁵ à 4 x 10⁶ pores/cm².

2. Dispositif de culture par séparation sur membrane selon la revendication 1, dans lequel la pluralité des structures supérieures ont des membranes de séparation ayant chacune une taille de pore différente et/ou une densité des pores différente.

3. Kit de culture par séparation sur membrane comprenant un dispositif de culture par séparation sur membrane selon l'une quelconque des revendications 1 et 2 et un ou plusieurs facteurs de migration cellulaire à verser dans la structure inférieure,
dans lequel le ou les facteurs de migration cellulaire sont un ou plusieurs choisis parmi SDF-1, G-CSF, bFGF, TGF-β, NGF, PDGF, BDNF, GDNF, EGF, VEGF, MMP3, Slit, GM-CSF, LIF, HGF et le sérum.

4. Kit selon la revendication 3, dans lequel la concentration du ou des facteurs de migration cellulaire est de 1 ng/ml à 500 ng/ml.

5. Kit selon la revendication 3, qui comprend en outre du sérum à verser dans la structure inférieure et dans lequel le facteur de migration cellulaire est G-CSF ou bFGF.

6. Procédé de séparation de cellules souches en utilisant le dispositif de culture par séparation sur membrane selon l'une quelconque des revendications 1 et 2, dans lequel le procédé comprend :
une étape consistant à disperser les cellules d'essai ou les tissus d'essai sur la membrane de séparation de la structure supérieure ;
une étape consistant à remplir le récipient en tant que structure inférieure d'un milieu contenant un ou plusieurs facteurs de migration cellulaire ; et
une étape consistant à laisser la membrane de séparation de la structure supérieure venir en contact avec le milieu dans la structure inférieure,
dans lequel le ou les facteurs de migration cellulaire sont un ou plusieurs choisis parmi SDF-1, G-CSF, bFGF, TGF-β, NGF, PDGF, BDNF, GDNF, EGF, VEGF, MMP3, Slit, GM-CSF, LIF, HGF et le sérum.

7. Procédé selon la revendication 6, dans lequel la concentration du ou des facteurs de migration cellulaire est de 1 ng/ml à 500 ng/ml.
